# EUROPÄISCHE PATENTANMELDUNG

(11) **EP 4 282 853 A1**
(43) Veröffentlichungstag der Anmeldung: **29.11.2023**
(21) Anmeldenummer: 23185347.4
(22) Anmeldetag: 27.09.2017
(51) Int. Cl.: C07C 67/12, C07C 69/593, C07C 51/56, C07C 57/13, C07C 67/333

(54) **VERFAHREN ZUR HERSTELLUNG VON ESTERN DER MESACONSÄURE**

(30) Priorität: 21.12.2016 EP 16205788
(62) Teilanmeldung aus: 17771776.6
(71) Anmelder: Symrise AG, 37603 Holzminden (DE)
(72) Erfinder: CHANDRASEKARAN, Vijayanand, 37603 Holzminden (DE); PANTEN, Johannes, 37671 Höxter (DE); KOCH, Oskar, 37079 Göttingen (DE); DIAZ GOMEZ, Edison, 38642 Goslar (DE)
(74) Vertreter: Global IP Europe Patentanwaltskanzlei

(57) **Zusammenfassung**

Bereitgestellt werden Riechstoffmischung und deren Anwendungen, insbesondere Parfümöle, kosmetische Mittel, Auftragungsmittel oder Wasch- und Reinigungsmittel, enthaltend eine sensorisch wirksame Menge von (i) (E)-2-Methyl-but-2-endicarbonsäure-diethylester, (ii) (Z)-2-Methyl-but-2-endicarbonsäure-diethylester oder (iii) 2-Methylenbutandicarbonsäurediethylester sowie deren Mischungen und von diesen Verbindungen und Mischungen abgeleitete analoge Ester.

## Beschreibung

Die Erfindung befindet sich auf dem Gebiet der Duftstoffe und betrifft Riechstoffmischungen mit fruchtiger, birnenähnlicher Note, Mittel, die die spezielle Duftstoffe oder Duftstoffmischungen enthalten, Verfahren zur Herstellung von speziellen Duftstoffen mit birnenähnlicher Note, ein Parfümierungsverfahren sowie die Verwendung spezieller Duftstoffe zur Erzeugung einer birnenähnlichen Duftnote.

Trotz einer Vielzahl bereits vorhandener Riechstoffe besteht in der Parfümindustrie auch weiterhin ein genereller Bedarf an neuen Riechstoffen. Insbesondere besteht ein Bedarf an Riechstoffen, die sich nicht nur durch neue originelle Duftnoten auszeichnen, sondern auch über ihre geruchlichen Eigenschaften hinaus zusätzliche positive Sekundäreigenschaften besitzen, wie z. B. eine höhere Stabilität und Ausgiebigkeit unter bestimmten Anwendungsbedingungen, ein besseres Haftungsvermögen, eine größere Ausstrahlungskraft, einen niedrigeren Schwellenwert oder aber auch bessere dermatologische und toxikologische Resultate, wie z.B. eine gute biologische Abbaubarkeit.

Insbesondere besteht ein Bedarf an Riechstoffen, die bei einer niedrigeren Dosierung einen gleichen oder höheren Duftbeitrag leisten als vergleichbare Substanzen und damit zu einem niedrigeren Eintrag in die Umwelt führen (low- volume - high impact). Oder die bei einem gleichen Duftbeitrag deutlich preiswerter zur Verfügung gestellt werden können. Hier besteht speziell ein Bedarf nach Duftstoffen mit komplexen Geruchseigenschaften, wie z.B. fruchtig birnen-artigen. Essigsäureamylester, der ansonsten für sein birnenartiges Aroma bekannt ist, erfüllt diese Bedingungen indes gerade nicht.

Die Aufgabe der vorliegenden hat daher darin bestanden, Riechstoffe und Parfümkompositionen mit birnenähnlichem Aroma zur Verfügung auf Basis nachwachsender Rohstoffe, vorzugsweise Abfallstoffen, zu stellen, die die genannten Nachteile des Stands der Technik überwinden, d.h. insbesondere schon in niedrigen Dosierungen einen hohen Duftbeitrag leisten, einfach und stabil in vielfältigen Parfümund Endproduktformulierungen eingearbeitet werden können und dabei mit geringem technischen Aufwand und zu niedrigen Kosten hergestellt werden können.

### ALLGEMEINE BESCHREIBUNG DER ERFINDUNG

Ein erster Gegenstand der Erfindung betrifft eine Riechstoffmischung, enthaltend eine sensorisch wirksame Menge an einer, zwei oder allen drei Verbindungen aus der Gruppe, die gebildet wird von: Formel (i), der Formel (ii), und der Formel (iii): 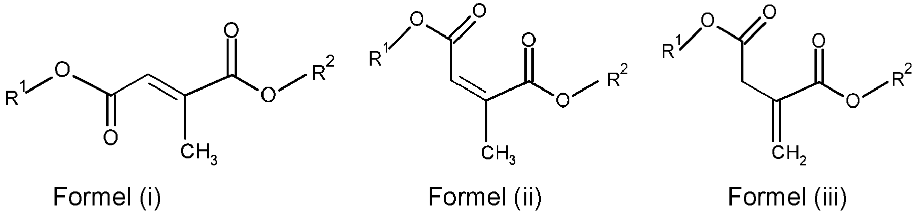 wobei R1 jeweils einen linearen, verzweigten oder cyclischen Alkylrest mit 1 bis 10 Kohlenstoffatomen, bevorzugt eine Methyl- oder Ethylgruppe, einen araliphatischen oder aromatischen Rest, darstellt, und wobei R2 jeweils einen linearen, verzweigten oder cyclischen Alkylrest mit 1 bis 10 Kohlenstoffatomen, bevorzugt eine Methyl- oder Ethylgruppe, einen araliphatischen oder aromatischen Rest darstellt.

Überraschenderweise wurde gefunden, dass sich insbesondere die Verbindungen der Formel (i) als birnen-artiger Riechstoff eignen und dabei das eingangs geschilderte komplexe Anforderungsprofil in vollem Umfang erfüllen. Die Stoffe der Formel (ii) und der Formel (iii) fallen bei der Herstellung mit an und weisen ebenfalls abgeschwächt ein birnen-artiges Aroma auf. Dabei ist insbesondere auch beachtlich, dass es sich hierbei um Substanzen handelt, die aus nachwachsenden Rohstoffen, speziell aus Abfällen der Zuckerindustrie gewonnen werden. Überraschend war ferner, dass sich diese Stoffe in eine Vielzahl unterschiedlicher Formulierung dauerhaft stabil einarbeiten lassen und dabei insbesondere den Standard der Ethyldecadienoate übertreffen.

Vorzugsweise sind R1 und R2 jeweils ein gleicher oder unterschiedlicher linearer, verzweigter oder cyclischer Alkylrest mit 1 bis 8 oder 1 bis 6 Kohlenstoffatomen, bevorzugt eine Methyl- oder Ethylgruppe. Die linearen Reste sind hierbei betont wertvoll, insbesondere bei Alkylresten mit 1 bis 4 Kohlenstoffatomen. Alternativ bevorzugt sind verzweigte Alkylreste mit dieser Kohlenstoffatomanzahl.

Die Gruppe der bevorzugten linearen Alkylreste für R1 und R2 mit 1 bis 10 Kohlenstoffatomen in den Verbindungen der Formel (i), der Formel (ii) und/oder der Formel (iii) wird gebildet aus den folgenden Radikalresten: Methyl, Ethyl, n-Propyl, n-Butyl, n-Pentyl, n-Hexyl, n-Heptyl, n-Octyl, n-Nonyl und n-Decyl. Bevorzugte lineare Alkylreste sind Methyl, Ethyl, aber ebenfalls geeignet sind n-Propyl, n-Butyl, n-Pentyl und n-Hexyl. Besonders bevorzugte lineare Alkylreste sind Methyl, Ethyl, n-Propyl und n-Butyl.

Die Gruppe der verzweigten Alkylreste für R1 und R2 mit 1 bis 10 Kohlenstoffatomen in den Verbindungen der Formel (i), der Formel (ii) und/oder der Formel (iii) wird gebildet aus: Isopropyl, Isobutyl, sec.-Butyl, tert.-Butyl, Isopentyl, tert.-Pentyl, iso-Hexyl, tert.-Hexyl, iso-Heptyl, iso-Octyl, tert.-Octyl, iso-Nonyl, tert.-Nonyl, tert.-Decyl und iso-Decyl. Bevorzugte verzweigte Alkylreste sind Isopropyl, Isobutyl, sec.-Butyl und tert.-Butyl. Besonders bevorzugte verzweigte Alkylreste sind Isopropyl und Isobutyl.

Die Gruppe der cyclischen Alkylreste für R1 und R2 mit 1 bis 10 Kohlenstoffatomen in den Verbindungen der Formel (i), der Formel (ii) und/oder der Formel (iii) wird gebildet aus: Cyclopropan, Cyclobutan, Cyclopentan, Cyclohexan, Cycloheptan und Cyclooctan. Bevorzugte cyclische Alkylreste sind Cyclopentan und Cyclohexan.

Unter der Gruppe der araliphatischen Reste für R1 und R2 in den Verbindungen der Formel (i), der Formel (ii) und/oder der Formel (iii) sind Alkylrest zu verstehen, an welchem ein oder mehrere Wasserstoffatome durch einen aromatischen Rest ersetzt sind. Die Gruppe der araliphatischen Reste in den Verbindungen der Formel (i), der Formel (ii) und/oder der Formel (iii) wird gebildet aus: Toluol, 2-Methylfuran, 3-Methylfuran, Picoline, Kresole, Xylole, 1-Methylnaphthalin, 2-Methylnaphthalin und Ethylbenzol. Bevorzugte araliphatische Reste sind Toluol und Ethylbenzol.

Die Gruppe der aromatischen Reste für R1 und R2 in den Verbindungen der Formel (i), der Formel (ii) und/oder der Formel (iii) wird gebildet aus: Pyrimidine, Furan, Thiran, Benzol, Naphthalin, Phenol, Pyridin und Benzodiazepine. Bevorzugte aromatische Reste sind Furan und Benzol.

Unter einer sensorisch wirksamen Menge ist dabei ein Anteil der Stoffe der Formel (i), der Formel (ii) und/oder der Formel (iii) in der Mischung zu verstehen, der ausreichend ist, um einen fruchtigen, birnenähnlichen Geruchseindruck hervorzurufen. Dieser fruchtige, birnenähnliche Geruchseindruck wird grundsätzlich hervorzurufen, wenn mindestens 0.001 Gew% an der Riechstoffmischung durch eine, zwei oder allen drei Verbindungen der Formel (i), der Formel (ii) und der Formel (iii) vorliegt.

In einer bevorzugten Weiterbildung der vorliegenden Erfindung ist der Rest R1 und/oder R2 in den Verbindungen der Formel (i), der Formel (ii) und der Formel (iii) in der oben beschriebenen Riechstoffmischung jeweils unabhängig voneinander ein C1 bis C4 Rest, d.h. insbesondere ein Methyl-, Ethyl-, Propyl-, und Butylrest. Diese Verbindungen sind besonders einfach herzustellen und zeigen die eindrucksvollsten Geruchsprofile.

In einer alternativen bevorzugten Weiterbildung der vorliegenden Erfindung ist der Rest R1 in den Verbindungen der Formel (i), der Formel (ii) und der Formel (iii) in der oben beschriebenen Riechstoffmischung jeweils unabhängig voneinander ein Methyl-, Ethyl-, Propyl-, und/oder Butylrest und der Rest R2 in den Verbindungen der Formel (i), der Formel (ii) und der Formel (iii) in der oben beschriebenen Riechstoffmischung jeweils unabhängig voneinander ein Methylrest oder Ethylrest.

In einer weiter bevorzugten Weiterbildung der vorliegenden Erfindung ist der Rest R2 in den Verbindungen der Formel (i), der Formel (ii) und der Formel (iii) in der oben beschriebenen Riechstoffmischung jeweils unabhängig voneinander ein Methyl-, Ethyl-, Propyl-, und Butylrest und der Rest R1 in den Verbindungen der Formel (i), der Formel (ii) und der Formel (iii) in der oben beschriebenen Riechstoffmischung jeweils unabhängig voneinander ein Methylrest oder Ethylrest.

In einer zweiten Variante der vorliegenden Erfindung sind die Reste R1 und R2 in den Verbindungen der Formel (i), der Formel (ii) und der Formel (iii) in der oben beschriebenen Riechstoffmischung jeweils dieselben Reste. Dies vereinfacht die Herstellung.

In einer dritten Variante der vorliegenden Erfindung sind die Reste R1 und R2 in den Verbindungen der Formel (i), der Formel (ii) und der Formel (iii) in der beschriebenen Riechstoffmischung jeweils unabhängig voneinander ausgewählt aus der Gruppe, die gebildet wird von: linearen oder verzweigten Methyl-, Ethyl-, Propyl-, und Butyl-Alkylresten. Gerade die linearen Alkylreste und kürzeren verzweigten Alkylreste sind geeignet, um in die Geruchsbindungstaschen zu passen.

In einer bevorzugten Weiterbildung der vorliegenden Erfindung sind die Reste R1 und R2 in den Verbindungen der Formel (i), der Formel (ii) und der Formel (iii) in der oben beschriebenen Riechstoffmischung jeweils unabhängig voneinander Methyl-, Ethyl-, Propyl-, und Butylreste.

In weiteren bevorzugten Weiterbildungen der vorliegenden Erfindung sind die Reste R1 und R2 in den Verbindungen der Formel (i), der Formel (ii) und der Formel (iii) in der oben beschriebenen Riechstoffmischung jeweils unabhängig voneinander Methyl-, und Propyl-Reste.

In einer vierten Variante betrifft die Erfindung eine Riechstoffmischung, enthaltend eine sensorisch wirksame Menge an einer, zwei oder allen drei Verbindungen aus der Gruppe, die gebildet wird von:
(i) (E)-2-Methyl-but-2-endicarbonsäure-diethylester,
(ii) (Z)-2-Methyl-but-2-endicarbonsäure-diethylster, und
(iii) 2-Methylenbutandicarbonsäurediethylester.

Diese drei Verbindungen (i), (ii) und (iii) stellen jeweils die besonders bevorzugten Varianten der erfindungsgemäßen Formeln der Riechstoffmischung dar, aber auch die Methylester oder Propylester sind günstig. Die Kombination von Ethylester, Methylester und/oder Propylester ist auch möglich.

RIECHSTOFFE: Bei den Riechstoffen der vorliegenden Erfindungen ist die Substanz (E)-2-Methyl-But-2-endicarbonsäure-diethylester der Formel (I) 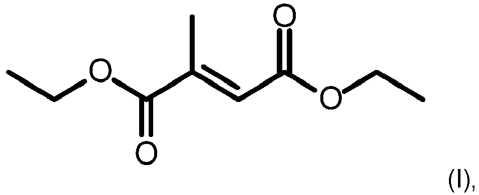 die auch als Mesaconsäurediethylester bezeichnet wird, für die Erfindung von besonderer Bedeutung und als solche bereits aus der Literatur bekannt. Hierzu wird auf die folgenden Zitierungen verwiesen: Advanced Synthesis & Catalysis, 2012, 354 (14-15), 2859-2864; Organic Letters, 2008; 10(21), 4815-4818; Organic & Biomolecular Chemistry, 2010, 8(19), 4444-4450; Journal of American Chemical Society, 2005, 127(15), 5518-5527; Tetrahedron Letters, 1996, 37(5), 629-632; Tetrahedron Letters, 1983, 39(9), 1475-1485; Journal of American Chemical Society, 2015, 137(26), 8556-8563; Organic Letters, 2011; 13(7), 1884-1887; Australian Journal of Chemistry, 1984, 37(2), 417-424; Tetrahedron Letters, 1981, 22(5), 381-384; Synthetic Communications, 1977, 7(6), 375-382; Helvetica Chimica Acta, 1974, 57(3), 856-863.

Im Folgenden werden die beiden Bezeichnungen (E)-2-Methyl-But-2-endicarbonsäure-diethylester und Mesaconsäurediethylester synonym verwendet. Bislang nicht bekannt waren die sensorischen Eigenschaften dieser Verbindung und insbesondere ihre Eignung als Duftstoff mit spezieller intensiver Birnennote. Diese Verwendung dieser Verbindung als Duftstoff ist daher ebenfalls Gegenstand dieser Erfindung.

Bei den beiden Verbindung der Formel (ii) und der Formel (iii) handelt es sich um Citraconsäurediethylester sowie Itaconsäurediethylester, die in der Regel zusammen mit dem Mesaconsäurediethylester als Isomerengemisch bei der Herstellung anfallen. Besonders bevorzugt ist für Verbindung (ii) die (Z)-2-Methyl-but-2-endicarbonsäure-diethylster und für Verbindung (iii) die 2-Methylenbutandicarbonsäurediethylester, welche mit dem Mesaconsäurediethylester (E)-2-Methyl-But-2-endicarbonsäure-diethylester ein bevorzugtes Verbindungsgemisch ergeben. Auch die Verwendung dieser Verbindungen einzelnd oder in Kombination mit Mesaconsäurediethylester als Riechstoffe oder Riechstoffgemische ist erfindungsgemäß bereitgestellt.

Bevorzugt sind Isomerengemische, die die Verbindung der Formel (i), vorzugsweise (E)-2-Methyl-But-2-endicarbonsäure-diethylester, in Mengen von mindestens 50 bis 100 Gew.-%, insbesondere etwa 70 bis etwa 90 Gew.-% und besonders bevorzugt von etwa 75 bis 85 Gew.-% aufweisen.

In einer fünften bevorzugten Weiterbildung der vorliegenden Erfindung, ist die Verbindung der Formel (i), bezogen auf die Summe der Verbindungen der Formel (i), der Formel (ii) und der Formel (iii), in einer Menge von 50 bis 100 Gew.-% in der Riechstoffmischung enthalten. Dies gilt auch für die besonders bevorzugten Verbindungen der jeweiligen Diethylester. Nach der Erfindung bevorzugt ist auch Mesaconsäurediethylester in mindestens 98.0 %-iger aber vor allem auch in 100.0%-iger Reinheit. Diese Riechstoffmischung mit reinem oder fast reinem Mesaconsäurediethylester zeigt einen besonders starken Geruch mit Birnennote.

In einer bevorzugten Variante der Erfindung ist die Verbindung der Formel (i) im Verhältnis zu der Summe aus den beiden Verbindungen der Formel (ii) und der Formel (iii) in der Riechstoffmisch mindestens 8 (Verbindung der Formel (i)) zu 1 (Summe aus den beiden Verbindungen der Formel (ii) und der Formel (iii)), besonders bevorzugt im Verhältnis 9:1 oder mit noch größerem Anteil der Formel (i) enthalten. Diese Verhältnisse gelten auch für die Verbindungsmischung von Mesaconsäurediethylester zu Citraconsäurediethylester und Itaconsäurediethylester. Bei höheren Anteilen der Mesaconsäureesteranteile entsteht ein stärkerer Birnengeruch.

Es wurde insbesondere gefunden, dass sich das Isomerengemisch von (i), (ii) und (iii) im Allgemeinen und die Mesaconsäureester der Verbindung (i), v.a. das Mesaconsäurediethylester, im Besonderen hervorragend zum Vermitteln, Modifizieren und/oder Verstärken eines birnen-artigen Duftes eignet. Die Tatsache, dass die Verbindungen einen solch aussagestarken Duft aufweisen, ist überraschend, da sie sich von bekannten Substanzen mit ähnlichen Strukturen in den oben beschriebenen Geruchseigenschaften unterscheidet. So weist z.B. Fumarsäurediethylester (II) ebenfalls eine Butendicarbonsäure-Struktur auf, wird jedoch als fast geruchslos beschrieben: (Quelle: Sicherheitsdatenblatt Fumarsäurediethylester, Fa. Merck):

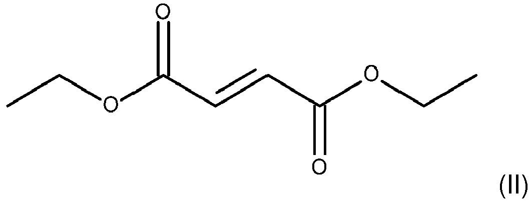

Geruchlich ähnliche Verbindungen hingegen, wie z.B. das 2,4-Ethyldecadienoate (III) weisen wiederum gänzlich andere Strukturen auf (vgl.: H. Surburg, J. Panten, Common Fragrance and Flavor Materials, 6th ed., Wiley-VCH, Weinheim, 2016, S.25):

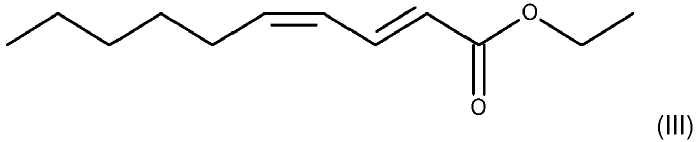

Dabei erweisen sich diese Stoffe zudem als deutlich schwächer, was Geruchsintensität und Haftung angeht.

Zu erwähnen sind ferner neben den besonderen olfaktorischen Vorteilen die hervorragenden stofflichen Eigenschaften, wie Löslichkeit in üblichen kosmetischen Lösungsmitteln, Kompatibilität mit weiteren Bestandteilen derartiger Produkte sowie die toxikologische Unbedenklichkeit der Verbindung, die die besondere Eignung der erfindungsgemäßen Verbindung für die genannten Einsatzzwecke unterstreichen. Ein weiterer Vorteil gegenüber den herkömmlichen Riechstoffen mit ähnlichen Geruchsprofilen (wie z.B. nach Formel III) liegt in seiner sehr günstigen Verfügbarkeit und Herstellung.

In einer sechsten bevorzugten Weiterbildung der vorliegenden Erfindung sind die Verbindungen der Formel (i), der Formel (ii) und/oder der Formel (iii), bezogen auf die Riechstoffmischung, in einer Menge von zusammen 0,001 bis 99,999 Gew.-% enthalten.

Noch bevorzugter sind die Verbindungen der Formel (i), der Formel (ii) und/oder der Formel (iii), bezogen auf die Riechstoffmischung, in einer Menge von zusammen 0,05 bis 50 Gew.-% enthalten. Dies gilt auch für die besonders bevorzugten Verbindungen der jeweiligen Diethylester.

In einer alternativ bevorzugten Ausführungsform der vorliegenden Erfindung sind die Verbindungen der Formel (i), der Formel (ii) und/oder der Formel (iii), bezogen auf die Riechstoffmischung, in einer Menge von zusammen 1 bis 30 Gew.-% enthalten.

In einer besonders bevorzugten Ausführungsform der vorliegenden Erfindung können die Mischungen die Verbindungen der Formel (i), der Formel (ii) und/oder der Formel (iii) im Allgemeinen, oder das (E)-2-Methyl-but-2-endicarbonsäure-diethylester für sich, bzw., das Mesaconsäurediethylester in Mischung mit Citraconsäurediethylester sowie Itaconsäurediethylester im Besonderen, in einer sensorisch wirksamen Menge von 0,001 bis etwa 99,999 Gew.-%, vorzugsweise in einer Menge von etwa 0,01 bis etwa 90 Gew.-%, bevorzugt in einer Menge von etwa 0,05 bis etwa 50 Gew.-% und besonders bevorzugt in einer Menge von etwa 0,5 bis etwa 25 Gew.-% enthalten. Die Summe zu 100 Gew.-% machen dann bei Riechstoffmischungen in der Regel die weiteren Duftstoffe sowie gegebenenfalls Lösungs- und/oder Konservierungsmittel aus. Je nach Anwendung können aber auch andere Zusätze hinzukommen.

**DUFTSTOFFE:** Die erfindungsgemäßen Riechstoffmischungen enthalten zwingend einen weiteren Duftstoff, d.h. mindestens zwei Duftstoffkomponenten, bzw. bestehen aus der Isomerenmischung der Verbindungen der Formel (i), der Formel (ii) und/oder der Formel (iii), und hierbei insbesondere (E)-2-Methyl-but-2-endicarbonsäure-diethylester in einer sensorisch wirksamen Menge mit mindestens einem weiteren Duftstoff. Üblicherweise werden Duftstoffe nicht in binären oder ternären Mischungen eingesetzt, sondern als Bestandteil ausgeklügelter komplexer Mischungen, die 10, 20, 50, 100 oder mehr Duftstoffe in zum Teil sehr geringen Mengen enthalten können, um ein besonders rundes Geruchsprofil zu ergeben. In diesem Sinne sind auch die vorliegenden Mischungen der Erfindung zu verstehen, nämlich als Mischung der Mischung aus den Verbindungen der Formel (i), der Formel (ii) und/oder der Formel (iii) mit einem, zwei, drei, vier, fünf, zehn, vorzugsweise aber einer noch weitaus höheren Zahl von Duftstoffen.

In einer siebten Weiterbildung der vorliegenden Erfindung enthält die beschriebene Riechstoffmischung eine beliebig große Anzahl weiterer Duftstoffe, die ausgewählt sind aus der Gruppe, die gebildet wird von: (1) Kohlenwasserstoffen; (2) Aliphatischen Alkoholen; (3) Aliphatischen Aldehyden und deren Acetalen; (4) Aliphatischen Ketonen und deren Oximen; (5) Aliphatischen schwefelhaltigen Verbindungen; (6) Aliphatischen Nitrilen; (7) Estern von aliphatischen Carbonsäuren; (8) Acyclischen Terpenalkoholen; (9) Acyclischen Terpenaldehyden und -ketonen; (10) Cyclischen Terpenalkoholen; (11) Cyclischen Terpenaldehyde und -ketonen; (12) Cyclischen Alkoholen; (13) Cycloaliphatischen Alkoholen; (14) Cyclischen und cycloaliphatischen Ethern; (15) Cyclischen und makrocyclischen Ketonen; (16) Cycloaliphatischen Aldehyden; (17) Cycloaliphatischen Ketonen; (18) Estern cyclischer Alkohole; (19) Estern cycloaliphatischer Alkohole; (20) Estern cycloaliphatischer Carbonsäuren; (21) Araliphatischen Alkoholen; (22) Estern von araliphatischen Alkoholen und aliphatischen Carbonsäuren; (23) Araliphatischen Ethern; (24) Aromatischen und araliphatischen Aldehyden; (25) Aromatischen und araliphatischen Ketonen; (26) Aromatischen und araliphatischen Carbonsäuren und deren Estern; (27) Stickstoffhaltigen aromatischen Verbindungen; (28) Phenolen, Phenylethern und Phenylestern; (29) Heterocyclischen Verbindungen; (30) Lactone; sowie deren Gemischen.

Die Auswahl der Duftstoffe ist dabei sehr umfassend; entsprechende Stoffe, mit denen Mesaconsäurediethylester vorteilhaft kombiniert werden kann, finden sich z. B. in "S. Arctander, Perfume and Flavor Chemicals, Vol. I und II, Montclair, N. J., 1969, Selbstverlag" oder "H. Surburg und J. Panten, Common Fragrance and Flavor Materials, 6th ed., Wiley-VCH, Weinheim, 2016". Im Einzelnen seien im Folgenden genannt:
**Extrakte aus natürlichen Rohstoffen:** Diese Gruppe steht für Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen.
**Einzel-Riechstoffe:** Einzelne Riechstoffe lassen sich in eine Vielzahl von Klassen gliedern, nämlich:
**Kohlenwasserstoffe,** wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
**Aliphatische Alkohole** wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
**Aliphatische Aldehyde und deren Acetale** wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
**Aliphatische Ketone und deren Oxime** wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
**Aliphatische schwefelhaltigen Verbindungen** wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
**Aliphatische Nitrile** wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
**Ester von aliphatischen Carbonsäuren** wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
**Acyclische Terpenalkohole** wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
**Acyclische Terpenaldehyde und -ketone** wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
**Cyclische Terpenalkohole** wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
**Cyclische Terpenaldehyde und -ketone** wie z. B. Menthon; Isomenthon ; 8-Mercaptomenthan-3-on ; Carvon; Campher; Fenchon; alpha-Ionon; beta-Ionon; alphan-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
**Cyclische Alkohole** wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
**Cycloaliphatische Alkohole** wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
**Cyclische und cycloaliphatische Ether** wie z.B. Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1 -yl)-5-methyl-5-(1 - methylpropyl)-1,3-dioxan;
**Cyclische und makrocyclische Ketone** wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1, 1,2,3,3-pentamethyl-4(5H)-indanon; 8-Cyclohexadecen-1-on; 9-Cycloheptadecen-1-on; Cyclopentadecanon; Cyclohexadecanon;
**Cycloaliphatische Aldehyde** wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
**Cycloaliphatische Ketone** wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
**Ester cyclischer Alkohole** wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
**Ester cycloaliphatischer Alkohole** wie z. B. 1 -Cyclohexylethylcrotonat;
**Ester cycloaliphatischer Carbonsäuren** wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
**Araliphatische Alkohole** wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
**Ester von araliphatischen Alkoholen und aliphatischen Carbonsäuren** wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
**Araliphatische Ether** wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
**Aromatische und araliphatische Aldehyde** wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
**Aromatische und araliphatische Ketone** wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon-2-Benzofuranylethanon; (3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
**Aromatische und araliphatische Carbonsäuren und deren Ester** wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
**Stickstoffhaltige aromatischen Verbindungen** wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
**Phenole, Phenylether und Phenylester** wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
**Heterocyclische Verbindungen** wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
**Lactone** wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin;
sowie deren beliebige Mischungen.

### ANWENDUNGSFORMEN

Ein achter Aspekt der vorliegenden Erfindung betrifft Parfümöle, kosmetische Mittel, Auftragungsmittel oder Wasch- und Reinigungsmittel, welche die beschriebene erfinderische Riechstoffmischung enthalten.

Unter einem Auftragungsmittel sind alle hierin genannten Mittel zu verstehen, die nicht unter die Gruppe der Parfümöle, kosmetischen Mittel, oder Wasch- und Reinigungsmittel fallen.

Bei diesen Mitteln ist nach einer neunten Weiterbildung der Erfindung die erfinderische Riechstoffmischung in Mengen von 0,05 bis 5 Gew.-%, bezogen auf das Mittel, enthalten. Die Erfindung betrifft daher sowohl Pafümöle, kosmetische Mittel, die Auftragungsmittel, als auch die Wasch- und Reinigungsmittel, einschließlich auch der parfümierten oder bedufteten Produkte, welche die Verbindungen der Formel (i), der Formel (ii) und/oder der Formel (iii) im Allgemeinen, oder das (E)-2-Methyl-but-2-endicarbonsäure-diethylester für sich, bzw., das Mesaconsäurediethylester in Mischung mit Citraconsäurediethylester sowie Itaconsäurediethylester im Besonderen, oder eine entsprechende Riechstoffmischung wie beschriebenen in Mengen von etwa 0,05 bis etwa 5 Gew.-%, bezogen auf das Mittel, vorzugsweise in Mengen von etwa 0,1 bis etwa 3 Gew.-% und insbesondere etwa 0,5 bis etwa 2 Gew.-% enthalten.

Riechstoffmischungen und Parfümöle, welche die Verbindungen der Formel (i), der Formel (ii) und/oder der Formel (iii), oder bevorzugt (E)-2-Methyl-but-2-endicarbonsäurediethylester, oder das Gemisch von Mesaconsäurediethylester, Citraconsäurediethylester und Itaconsäurediethylester enthalten, können in flüssiger Form, unverdünnt oder mit einem Lösungsmittel verdünnt, für Parfümierungen eingesetzt werden. Geeignete Lösungsmittel sind z. B. Ethanol, Isopropylalkohol, Diethylenglykolmonoethylether, Glycerin, Propylenglycol, 1,2-Butylenglycol, Dipropylenglykol, Diethylphthalat, Triethylcitrat, Isopropylmyristat usw.

Diese Riechstoffmischungen können dabei bis zu 90 Gew.-%, vorzugsweise etwa 5 bis etwa 70 Gew.-%, insbesondere etwa 10 bis etwa 50 Gew.-% und besonders bevorzugt etwa 15 bis etwa 25 Gew.-% der genannten Lösungsmittel enthalten.

Für manche Anwendungen ist es auch vorteilhaft, Parfümöle (Riechstoffmischungen) enthaltend Verbindungen der Formel (i), der Formel (ii) und/oder der Formel (iii), oder bevorzugt (E)-2-Methyl-but-2-endicarbonsäurediethylester, oder das Gemisch von Mesaconsäurediethylester, Citraconsäurediethylester und Itaconsäurediethylester an einem Trägerstoff adsorbiert einzusetzen, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulosebasierende Stoffe, Zucker oder Kunststoffe wie PVC, Polyvinylacetate oder Polyurethane sein.

Für andere Anwendungen ist es vorteilhaft, die Parfümöle bzw. Riechstoffmischungen der Erfindung mikroverkapselt, sprühgetrocknet, als Einschlusskomplex oder als Extrusionsprodukt einzusetzen und in dieser Form dem zu parfümierenden (Vor-)Produkt hinzufügen.

Die Eigenschaften derart modifizierter Parfümöle bzw. Riechstoffmischungen werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z.B. Polyvinylalkohol verwendet werden.

Die Mikroverkapselung der Parfümöle bzw. Riechstoffmischungen der Erfindung kann beispielsweise durch das sogenannte Koazervationsverfahren mit Hilfe von Kapselmaterialien z.B. aus polyurethanartigen Stoffen oder Weichgelatine erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschlusskomplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusionsprodukte können durch Verschmelzen der Parfümöle mit einem geeigneten wachsartigen Stoff und durch Extrusion mit nachfolgender Erstarrung, gegebenenfalls in einem geeigneten Lösungsmittel, z.B. Isopropylalkohol erfolgen.

### KOSMETISCHE MITTEL UND KÖRPERPFLEGEMITTEL

Die erfindungsgemäßen kosmetischen Mittel und Körperpflegemittel können neben den Verbindungen der Formel (i), der Formel (ii) und/oder der Formel (iii), oder bevorzugt *(E)*-2-Methyl-but-2-endicarbonsäurediethylester, oder dem Gemisch von Mesaconsäurediethylester, Citraconsäurediethylester und Itaconsäurediethylester, bzw. den diese Verbndungen oder Gemische enthaltenden Riechstoffmischungen weitere typische Hilfs- und Zusatzstoffe enthalten, wie beispielsweise die unten genannten milden Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Die Begriffe "kosmetische Mittel", "Körperpflegemittel" und "parfümierte Artikel" werden untereinander synonym verwendet und setzen nur voraus, dass die Mittel alle eine sensorisch wirksame Menge an den Verbindungen der Formel (i), der Formel (ii) und/oder der Formel (iii) oder bevorzugt (E)-2-Methyl-but-2-endicarbonsäurediethylester, oder das Gemisch von Mesaconsäurediethylester, Citraconsäurediethylester und Itaconsäurediethylester, bzw. einer diesen Diestern enthaltenden Riechstoffmischung aufweisen. Bevorzugte Zusatzstoffe werden im Folgenden genannt.

### Tenside:

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper:

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C6-C22-Fettsäuren mit verzweigten Alkoholen, insbesondere 2-Ethylhexanol, Ester von C18-C38-Alkylhydroxycarbonsäuren mit linearen oder verzweigten C6-C22-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, Ester von C2-C12-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C6-C22-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C6-C22-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

**Emulgatoren:** Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:
**Alkoxylate:** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C12/18-Fettsäuremono- und -diester von Anlagerungsprodukten von Ethylenoxid an Glycerin sind als Rückfettungsmittel für kosmetische Zubereitungen bekannt.
**Alkyl- und/oder Alkenyloligoglykosid:** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
**Partialglyceride:** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
**Sorbitanester:** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische in Frage. Ebenfalls geeignet sind Anlagerungsprodukte von 1 bis 30, vorzugsweise 5 bis 10 Mol Ethylenoxid an die genannten Sorbitanester.
**Polyglycerinester:** Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
**Anionische Emulgatoren:** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
**Amphotere und kationische Emulgatoren:** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oderAcylgruppe im Molekül mindestens eine freie Aminogruppe und mindestens eine -COOH- oder -SOsH-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.
**Fette und Wachse:** Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.
**Perlglanzwachse:** Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen
**Kühlstoffe:** Kühlstoffe sind Verbindungen, die auf der Haut ein Gefühl der Kälte erzeugen. In der Regel handelt es sich dabei um Mentholverbindungen, die - neben dem Grundkörper Menthol selbst - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen. FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

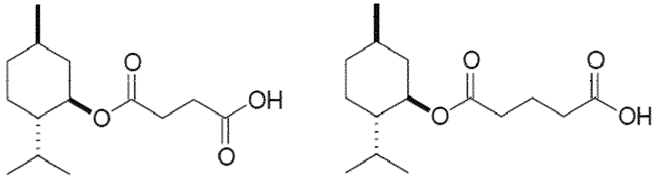

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

**Konsistenzgeber und Verdickungsmittel:** Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

**Überfettungsmittel und Stabilisatoren:** Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

**Polymere:** Geeignete kationische Polymere sind beispielsweise kationische Cellulosederivate, wie z.B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium Hydroxypropyl Hydrolyzed Collagen (Lamequat^{®}L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine^{®}/Sandoz), Copolymere der Acrylsäure mit Dimethyl-diallylammoniumchlorid (Merquat^{®} 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäurean-hydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

**Silikonverbindungen:** Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

**UV-Lichtschutzfaktoren:** Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
- Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyl-ester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan^{®} AP)
- Sulfonsäurederivate von Benzophenonen, vorzugsweise 2-Hydroxy-4-methoxybenzo-phenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE^{®} oder Z-COTE HP1^{®} verwendet.

**Feuchthaltemittel:** Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/- derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

**Biogene Wirkstoffe und Antioxidantien:** Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.
Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSOa) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

**Deodorantien und keimhemmende Mittel:** Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Keimhemmende Mittel:** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**Enzyminhibitoren:** Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**Geruchsabsorber:** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien:** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

**Filmbildner:** Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

**Antischuppenwirkstoffe:** Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

**Quellmittel:** Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

**Insektenrepellentien:** Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

**Inhaltsstoffe für Mund- und Zahnpflegemittel:** Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol^{®}-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p-Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süßungsmittel, wie z. B. Saccharin-Natrium, Natrium-Cyclamat, Sucrose, Lactose, Maltose, Fructose oder Apartam^{®}, (L-Aspartyl- L-phenylalaninmethylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

**Hydrotrope:** Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

**Konservierungsmittel:** Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

**Farbstoffe:** Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### WASCH- UND REINIGUNGSMITTEL

Die die Verbindungen der Formel (i), der Formel (ii) und/oder der Formel (iii), oder bevorzugt (E)-2-Methyl-but-2-endicarbonsäurediethylester, oder das Gemisch von Mesaconsäurediethylester, Citraconsäurediethylester und Itaconsäurediethylester bzw. die erfinderischen Riechstoffmischungen enthaltenden Wasch- und Reinigungsmittel (abgekürzt als WSR-Mittel) im Sinne der vorliegenden Erfindung, können in fester Form als Pulver, Granulate, Tabletten und dergleichen, aber auch flüssig, gelförmig oder pastös vorliegen. Es handelt es sich vorzugsweise um Waschmittel, die sowohl für manuelle oder maschinelle Wäsche, insbesondere von Textilien geeignet sind. Es kann sich auch um Wasch- oder Reinigungsmittel für den industriellen Bereich oder für den Haushaltsbereich handeln. Reinigungsmittel können beispielsweise auch zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Als harte Oberflächen kommen neben Geschirr auch alle übrigen harten Oberflächen, insbesondere aus Glas, Keramik, Kunststoff oder Metall, in Haushalt und Gewerbe in Frage.

Die WSR-Mittel können weitere handelsübliche Bestandteile aufweisen, wie beispielsweise Tenside, Gerüststoffe, Bleichmittel, Bleichmittelaktivatoren, Verdickungsmittel, Enzyme, Elektrolyte, pH-Stellmittel, Farb- und Duftstoffe, Schauminhibitoren, Antiredepositionsmittel, optische Aufheller, Vergrauungsinhibitoren, Knitterschutzmittel, antimikrobielle Wirkstoffe, Konservierungsmittel, Antioxidantien, Antistatika, UV-Adsorber, Schwermetallkomplexbildner und dergleichen. Diese Hilfsstoffe werden im Folgenden näher beschrieben:
**Tenside:** Als Tenside zur Herstellung der Wasch- oder Reinigungsmittel können neben den nichtionischen Tensiden auch Anion-, Kation-, Ampho- und/oder Niotenside und verzweigte Alkylsulfaten verwendet werden.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-14-Alkohole mit 3 EO, 4 EO oder 7 EO, C9-11-Alkohol mit 7 EO, C13-5-Alkohole mit 3 EO, 5 EO, 7 EO oder 8 EO, C12-18-Alkohole mit 3 EO, 5 EO oder 7 EO und Mischungen aus diesen, wie Mischungen aus C12-4-Alkohol mit 3 EO und C12-8-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Eine weitere Klasse von nichtionischen Tensiden, die vorteilhaft zur Herstellung von Wasch- oder Reinigungsmitteln eingesetzt werden kann, sind die Alkylpolyglycoside (APG). Einsetzbare Alkypolyglycoside genügen der allgemeinen Formel RO(G)Z, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können zur Herstellung der Wasch- oder Reinigungsmittel geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel R-CO-N(R1)-[Z], in der RCO für einen aliphatischen Acylrest mit 6 bis 22 Kohlenstoffatomen, R1 für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Zur Gruppe der Polyhydroxyfettsäureamide gehören auch Verbindungen der Formel R-CO-N(R1-O-R2)- [Z], in der R für einen linearen oder verzweigten Alkyl- oder Alkenylrest mit 7 bis 12 Kohlenstoffatomen, R1 für einen linearen, verzweigten oder cyclischen Alkylrest oder einen Arylrest mit 2 bis 8 Kohlenstoffatomen und R2 für einen linearen, verzweigten oder cyclischen Alkylrest oder einen Arylrest oder einen Oxy-Alkylrest mit 1 bis 8 Kohlenstoffatomen steht, wobei C1-4-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxysubstituierten Verbindungen können dann durch Umsetzung mit Fettsäuremethylestern in Gegenwart eines Alkoxids als Katalysator in die gewünschten Polyhydroxyfettsäureamide überführt werden.

Der Gehalt an nichtionischen Tensiden beträgt den flüssigen Wasch- und Reinigungsmitteln bevorzugt 5 bis 30 Gew.-%, vorzugsweise 7 bis 20 Gew.% und insbesondere 9 bis 15 Gew.%, jeweils bezogen auf das gesamte Mittel.

Als anionische Tenside werden beispielsweise solche vom Typ der Sulfonate und Sulfate eingesetzt. Als Tenside vom Sulfonat-Typ kommen dabei vorzugsweise C9-3-Alkylbenzolsulfonate, Olefinsulfonate, d.h. Gemische aus Alken- und Hydroxyalkansulfonaten sowie Disulfonaten, wie man sie beispielsweise aus C12-8-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C12-8-Alkanen beispielsweise durch Sulfochlorierung oder Sulfoxidation mit anschließender Hydrolyse bzw. Neutralisation gewonnen werden. Ebenso sind auch die Ester von alpha-Sulfofettsäuren (Estersulfonate), zum Beispiel die alphasulfonierten Methylester der hydrierten Kokos-, Palmkern- oder Talgfettsäuren geeignet.

Auch geeignet sind Sulfonierungsprodukte von ungesättigten Fettsäuren, beispielsweise Ölsäure, in geringen Mengen, vorzugsweise in Mengen nicht oberhalb etwa 2 bis 3 Gew.-%. Insbesondere sind alpha -Sulfofettsäurealkylester bevorzugt, die eine Alkylkette mit nicht mehr als 4 C-Atomen in der Estergruppe aufweisen, beispielsweise Methylester, Ethylester, Propylester und Butylester. Mit besonderem Vorteil werden die Methylester der alpha -Sulfofettsäuren (MES), aber auch deren verseifte Disalze eingesetzt.

Als weitere anionische Tenside kommen Fettsäure-Derivate von Aminosäuren, beispielsweise von N-Methyltaurin (Tauride) und/oder von N-Methylglycin (Sarkoside) in Betracht. Insbesondere bevorzugt sind dabei die Sarkoside bzw. die Sarkosinate und hier vor allem Sarkosinate von höheren und gegebenenfalls einfach oder mehrfach ungesättigten Fettsäuren wie Oleylsarkosinat.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C12-C18-Fettalkohole, beispielsweise aus Kokosfettalkohol, Talgfettalkohol, Lauryl-, Myristyl-, Cetyl- oder Stearylalkohol oder der C10-C20-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C12-C16-Alkylsulfate und C12-C15-Alkylsulfate sowie C14-C15-Alkylsulfate bevorzugt. Auch 2,3-Alkylsulfate, die beispielsweise als Handelsprodukte der Shell Oil Company unter dem Namen DAN(R) erhalten werden können, sind geeignete Aniontenside.

Auch die Schwefelsäuremonoester der mit 1 bis 6 Mol Ethylenoxid ethoxylierten geradkettigen oder verzweigten C7-21-Alkohole, wie 2-Methylverzweigte C9-11-Alkohole mit im Durchschnitt 3,5 Mol Ethylenoxid (EO) oder C12-18-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C8-18-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Der Gehalt bevorzugter flüssiger Wasch- und Reinigungsmittel an anionischen Tensiden beträgt 1 bis 30 Gew.%, vorzugsweise 4 bis 25 Gew.% und insbesondere 5 bis 22 Gew.%, jeweils bezogen auf das gesamte Mittel. Es ist besonders bevorzugt, dass die Menge an Fettsäureseife mindestens 2 Gew.% und besonders bevorzugt mindestens 3 Gew.% und insbesondere bevorzugt mindestens 4 Gew.% beträgt.

Als weitere Tenside kommen zur Herstellung der erfindungsgemäßen Wasch- oder Reinigungsmittel sogenannte Gemini-Tenside in Betracht. Hierunter werden im Allgemeinen solche Verbindungen verstanden, die zwei hydrophile Gruppen und zwei hydrophobe Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, dass die hydrophilen Gruppen einen ausreichenden Abstand haben, damit sie unabhängig voneinander agieren können. Derartige Tenside zeichnen sich im Allgemeinen durch eine ungewöhnlich geringe kritische Micellkonzentration und die Fähigkeit, die Oberflächenspannung des Wassers stark zu reduzieren, aus. In Ausnahmefällen werden jedoch unter dem Ausdruck Gemini-Tenside nicht nur dimere, sondern auch trimere Tenside verstanden.

Gemini-Tenside zur Herstellung von Wasch- oder Reinigungsmitteln sind beispielsweise sulfatierte Hydroxymischether gemäß der deutschen Patentanmeldung DE-A-43 21 022 oder Dimeralkoholbis- und Trimeralkohol-tris-sulfate und -ethersulfate gemäß der deutschen Patentanmeldung DE-A- 195 03 061. Endgruppenverschlossene dimere und trimere Mischether gemäß der deutschen Patentanmeldung DE-A-195 13 391 zeichnen sich insbesondere durch ihre Bi- und Multifunktionalität aus. So besitzen die genannten endgruppenverschlossenen Tenside gute Netzeigenschaften und sind dabei schaumarm, so dass sie sich insbesondere für den Einsatz in maschinellen Wasch- oder Reinigungsverfahren eignen.

Bevorzugt sind aus anwendungstechnischer Sicht Mischungen aus anionischen und nichtionischen Tensiden. Der Gesamt-Tensidgehalt des flüssigen Wasch- und Reinigungsmittels liegt vorzugsweise unterhalb von 40 Gew.- % und besonders bevorzugt unterhalb von 35 Gew.-%, bezogen auf das gesamte flüssige Wasch- und Reinigungsmittel.

**Gerüststoffe:** Als Gerüststoffe bzw. Builder, die in den flüssigen Wasch- und Reinigungsmitteln enthalten sein können, sind insbesondere Silikate, Aluminiumsilikate (insbesondere Zeolithe), Carbonate, organische Co-builder, Phosphate, Salze organischer Di- und Polycarbonsäuren sowie Mischungen dieser Stoffe zu nennen.

Geeignete kristalline, schichtförmige Natriumsilikate besitzen die allgemeine Formel NaMSiₓO₂ₓ₊₁*H2O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl beta - als auch delta -Natriumdisilikate Na₂Si₂O₅*yH₂O bevorzugt.

Einsetzbar sind auch amorphe Natriumsilikate mit einem Modul Na₂O: SiO₂ von 1: 2 bis 1: 3,3, vorzugsweise von 1: 2 bis 1: 2,8 und insbesondere von 1: 2 bis 1: 2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharte Beugungsmaxima liefern. Dies ist so zu interpretieren, dass die Produkte mikrokristalline Bereiche der Größe 10 bis einige Hundert nm aufweisen, wobei Werte bis maximal 50 nm und insbesondere bis maximal 20 nm bevorzugt sind. Derartige sogenannte röntgenamorphe Silikate, weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Ein verwendbarer feinkristalliner, synthetischer und gebundenes Wasser enthaltender Zeolith ist vorzugsweise Zeolith A und/oder P. Als Zeolith P wird Zeolith MAP TM (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma SASOL unter dem Markennamen VEGOBOND AX(R) vertrieben wird und durch die Formel:

nNa₂O*(1-n)K₂O*Al₂O₃.(2-2,5)*SiO₂.(3,5-5,5)*H₂O,

beschrieben werden kann, entspricht. Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Messmethode: Coulter Counter) auf und enthalten vorzugsweise 18 bis 22 Gew.%, insbesondere 20 bis 22 Gew.% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Als Builder sind organische Co-builder geeignet, insbesondere Polycarboxylate/Poly-carbonsäuren, polymere Polycarboxylate, Asparaginsäure, Polyacetale, Dextrine, sowie Phosphonate.

Polymere Polycarboxylate sind beispielsweise die Alkalimetallsalze der Polyacrylsäure oder der Polymethacrylsäure, beispielsweise solche mit einer relativen Molekülmasse von 500 bis 70000 g/mol. Bei den für polymere Polycarboxylate angegebenen Molmassen handelt es sich im Sinne dieser Schrift um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsaeure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2.000 bis 70.000 g/mol, vorzugsweise 20.000 bis 50.000 g/mol und insbesondere 30.000 bis 40.000 g/mol.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsaeure sowie Zucker-Derivate enthalten.

Weitere bevorzugte Copolymere sind solche, die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Co-builder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500.000 g/mol. Dabei ist ein Polysaccharid mit einem Dextrose-äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weissdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C6 des Saccharidrings oxidiertes Produkt kann besonders vorteilhaft sein.

Ein bevorzugtes Dextrin ist in der britischen Patentanmeldung GB 9,419,091 B1 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen EP 032202 A**,** EP 0427349 A**,** EP 0472042 A und EP 0542496 A sowie den internationalen Patentanmeldungen WO 1992/018542 A**,** WO 1993/008251 A**,** WO 1994/ 028030 A**,** WO 1995/007303 A, WO 1995/012619 A und WO 1995/020608 A bekannt. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate, wie sie beispielsweise in den USamerikanischen Patentschriften US 4,524,009**,** US 4,639 325**,** in der europäischen Patentanmeldung EP 0150930 A und der japanischen Patentanmeldung JP 1993/339896 A beschrieben werden.

Weitere brauchbare organische Co-builder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Co-builder werden beispielsweise in der internationalen Patentanmeldung WO 1995/020029 A beschrieben.

Eine weitere Substanzklasse mit Co-buildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-builder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Wasch- und Reinigungsmittel auch Bleiche enthalten, bevorzugt sein Aminoalkanphosphonate, insbesondere DTPMP, einzusetzen, oder Mischungen aus den genannten Phosphonaten zur Herstellung der Mittel zu verwenden.

Darüber hinaus können alle Verbindungen, die in der Lage sind, Komplexe mit Erdalkaliionen auszubilden, als Co-builder eingesetzt werden.

Weitere brauchbare organische Gerüstsubstanzen sind auch die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- und/oder Reinigungsmitteln. Insbesondere sind hierbei Citronensäure, Bernsteinsäure, Glutarsäure, Adipinsäure, Gluconsäure und beliebige Mischungen aus diesen zu nennen.

**Bleichmittel und Bleichkatalysatoren:** Unter den als Bleichmitteln dienenden, in Wasser H2O2 liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H2O2 liefernde persaure Salze oder Persäuren, wie Perbenzoate, Peroxophthalate, Diperazelainsäure, Phthaloiminopersäure oder Diperdodecandisäure. Um beim Waschen bei Temperaturen von 60 °C und darunter eine verbesserte Bleichwirkung zu erreichen, können Bleichaktivatoren in die Wasch-und Reinigungsmittel eingearbeitet werden. Als Bleichaktivatoren können Verbindungen, die unter Perhydrolysebedingungen aliphatische Peroxocarbonsäuren mit vorzugsweise 1bis 10 C-Atomen, insbesondere 2 bis 4 C-Atomen, und/oder gegebenenfalls substituierte Perbenzoesäure ergeben, eingesetzt werden. Geeignet sind Substanzen, die O-und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin(DADHT), acylierte Glykolurile, insbesondere Tetraacetylglykoluril (TAGU), N-Acylimide,insbesondere N-Nonanoylsuccinimid (NOSI), acylierte Phenolsulfonate, insbesonderen-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran. Zusätzlich zu den konventionellen Bleichaktivatoren oder an deren Stelle können auch sogenannte Bleichkatalysatoren in die Textilbehandlungsmitteleingearbeitet werden. Bei diesen Stoffen handelt es sich um bleichverstärkende Übergangsmetallsalze bzw. Übergangsmetallkomplexe wie beispielsweise Mn-, Fe-, Co-, Ru- oder Mo-Salenkomplexe oder -carbonylkomplexe. Auch Mn-, Fe-, Co-, Ru-, Mo-, Ti-, V- und Cu-Komplexe mit stickstoffhaltigen Tripod-Liganden sowie Co-, Fe-, Cu- und Ru-Amminkomplexe sind als Bleichkatalysatoren verwendbar.

**Verdickungsmittel:** Ein flüssiges Wasch- und Reinigungsmittel kann ein Verdickungsmittelenthalten. Das Verdickungsmittel kann beispielsweise einen Polyacrylat-Verdicker, Xanthan Gum, Gellan Gum, Guarkernmehl, Alginat, Carrageenan, Carboxymethylcellulose, Bentonite, Wellan Gum, Johannisbrotkernmehl, Agar-Agar, Tragant, Gummi arabicum, Pektine, Polyosen, Stärke, Dextrine, Gelatine und Casein umfassen. Aber auch abgewandelte Naturstoffe wie modifizierten Stärken und Cellulosen, beispielhaft seien hier Carboxymethylcellulose und andere Celluloseether, Hydroxyethyl- und - propylcellulose sowie Kernmehlether genannt, können als Verdickungsmittel eingesetzt werden.

Zu den Polyacryl- und Polymethacryl-Verdickern zählen beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung gemäß "International Dictionary of Cosmetic Ingredients "der "The Cosmetic, Toiletry and Fragrance Association (CTFA)": Carbomer), die auch als Carboxyvinylpolymere bezeichnet werden. Solche Polyacrylsäuren sind u.a. von der Fa. 3V Sigma unter dem Handelsnamen Polygel^{®},z.B. Polygel DA, und von der Fa. B.F. Goodrich unter dem Handelsnamen Carbopol^{®} erhältlich, z.B. Carbopol 940 (Molekulargewicht ca. 4.000.000), Carbopol 941 (Molekulargewicht ca. 1.250.000) oder Carbopol 934 (Molekulargewicht ca. 3.000.000).Weiterhin fallen darunter folgende Acrylsäure-Copolymere: (i) Copolymere von zwei oder mehr Monomeren aus der Gruppe der Acrylsäure, Methacrylsäure und ihrer einfachen, vorzugsweise mit C1-4-Alkanolen gebildeten, Ester (INCI AcrylatesCopolymer), zu denen etwa die Copolymere von Methacrylsäure, Butylacrylat und Methylmethacrylat (CAS-Bezeichnung gemäß Chemical Abstracts Service: 25035-69-2)oder von Butylacrylat und Methylmethacrylat (CAS 25852-37-3) gehören und die beispielsweise von der Fa. Rohm and Haas unter den Handelsnamen Aculyn^{®} und Acusol^{®} sowie von der Firma Degussa (Goldschmidt) unter dem Handelsnamen Tego^{®} Polymer erhältlich sind, z.B. die anionischen nicht assoziativen Polymere Aculyn 22, Aculyn 28, Aculyn 33 (vernetzt), Acusol 810,Acusol 820, Acusol 823 und Acusol 830 (CAS 25852-37-3); (ii) vernetzte hochmolekulare Acrylsäurecopolymere, zu denen etwa die mit einem Allylether der Saccharose oder des Pentaerythrits vernetzten Copolymere von C10-30-Alkylacrylaten mit einem oder mehreren Monomeren aus der Gruppe der Acrylsäure, Methacrylsäure und ihrer einfachen, vorzugsweise mit C1-4-Alkanolen gebildeten, Ester (INCI Acrylates/C10-30Alkyl Acrylate Crosspolymer) gehören und die beispielsweise von der Fa. B.F. Goodrich unter dem Handelsnamen Carbopol^{®} erhältlich sind, z.B. das hydrophobierte Carbopol ETD 2623 und Carbopol 1382 (INCI Acrylates/C10-30Alkyl Acrylate Crosspolymer) sowie Carbopol Aqua 30 (früher Carbopol EX 473).

Ein weiteres bevorzugt einzusetzendes polymeres Verdickungsmittel ist Xanthan Gum, ein mikrobielles anionisches Heteropolysaccharid, das von Xanthomonascampestris und einigen anderen Species unter aeroben Bedingungen produziert wird und eine Molmasse von 2 bis 15 Millionen Dalton aufweist. Xanthan wird aus einer Kette mit beta-1,4-gebundener Glucose (Cellulose) mit Seitenketten gebildet. Die Struktur der Untergruppen besteht aus Glucose, Mannose, Glucuronsaeure Acetat und Pyruvat, wobei die Anzahl der Pyruvat-Einheiten die Viskosität des Xanthan Gums bestimmt. Als Verdickungsmittel kommt insbesondere auch ein Fettalkohol in Frage. Fettalkohole können verzweigt oder nichtverzweigt sowie nativen Ursprungs oderpetrochemischen Ursprungs sein. Bevorzugte Fettalkohole haben eine C-Kettenlänge von 10 bis 20 C-Atomen, bevorzugt 12 bis 18. Bevorzugt werden Mischungen unterschiedlicher C-Kettenlängen, wie Talgfettalkohol oder Kokosfettalkohol, eingesetzt. Beispiele sind Lorol^{®} Spezial (C12-14-ROH) oder Lorol^{®} Technisch(C12-18-ROH) (beide ex Cognis). Bevorzugte flüssige Wasch- und Reinigungsmittel enthalten bezogen auf das gesamte Mittel 0.01 bis 3 Gew.% und vorzugsweise 0.1 bis1 Gew.% Verdickungsmittel. Die Menge an eingesetztem Verdickungsmittel ist dabei abhängig von der Art des Verdickungsmittels und dem gewünschten Grad der Verdickung.

**Enzyme:** Die Wasch- und Reinigungsmittel können Enzyme in verkapselter Form und/oder direkt in den Wasch- und Reinigungsmittel enthalten. Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen, Hemicellulase, Cutinasen, beta-Glucanasen, Oxidasen, Peroxidasen, Perhydrolasen und/oder Laccasen und Gemische der genannten Enzyme in Frage. Alle diese Hydrolasen tragen in der Wäsche zur Entfernung von Verfleckungen wie Protein-, fett- oder stärkehaltigen Verfleckungen und Vergrauungen bei. Cellulasen und andere Glykosylhydrolasenkoennen darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicolainsolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen, Iso-Amylasen, Pullulanasen und Pektinasen. Als Cellulasen werden vorzugsweise Cellobiohydrolasen, Endoglucanasen und p-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert sein, um sie gegen vorzeitige Zersetzung zu schützen. Der Anteil der Enzyme, der Enzymflüssigformulierung(en) oder der Enzymgranulate direkt in Wasch- und Reinigungsmittel kann beispielsweise etwa 0,01 bis 5 Gew.%, vorzugsweise 0.12 bis etwa 2.5 Gew.% betragen.

Es kann, beispielsweise bei speziellen Wasch- und Reinigungsmitteln für Konsumenten mit Allergien, aber auch bevorzugt sein, dass das Wasch- und Reinigungsmittel keine Enzyme enthält.

**Elektrolyte:** Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den Wasch- und Reinigungsmitteln bevorzugt. Der Anteil an Elektrolyten in den Wasch-und Reinigungsmitteln beträgt üblicherweise 0,1 bis 5 Gew.-%.

**Lösungsmittel:** Nichtwässrige Lösungsmittel, die in den flüssigen Wasch-und Reinigungsmitteln eingesetzt werden können, stammen beispielsweise aus der Gruppe der ein- oder mehrwertigen Alkohole, Alkanolamine oder Glykolether, sofern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethyl-, -ethyl-oder -propylether, Dipropylenglykolmonomethyl- oder -ethylether, Diisopropylenglykolmonomethyl-oder -ethylether, Methoxy-, Ethoxy- oder Butoxytriglykol, 1-Butoxyethoxy-2-propanol,3-Methyl-3-methoxybutanol, Propylenglykol-t-butylether sowie Mischungen dieser Lösungsmittel. Nichtwässrige Lösungsmittel können in den flüssigen Wasch- und Reinigungsmitteln in Mengen zwischen 0,5 und 15 Gew.%, bevorzugt aber unter 12 Gew.% und insbesondere unterhalb von 9 Gew.% eingesetzt werden.

**Viskositätsregulatoren:** Die Viskosität der Wasch- und Reinigungsmittel in flüssiger Form kann mit üblichen Standardmethoden (beispielsweise Brookfield-Viskosimeter LVT-II bei 20 U/min und 20°C, Spindel 3) gemessen werden und liegt für flüssige Waschmittel vorzugsweise im Bereich von 500 bis 5000 mPas. Bevorzugte weisen flüssige Wasch- und Reinigungsmitteln Viskositäten von 700 bis 4000 mPas auf, wobei Werte zwischen 1000 und 3000 mPas besonders bevorzugt sind. Die Viskosität von Weichspülern beträgt vorzugsweise 20 bis 4000 mPas, wobei Werte zwischen 40 und 2000 mPas besonders bevorzugt sind. Insbesondere bevorzugt liegt die Viskosität von Weichspülern von 40 bis 1000 mPas.

**pH-Stellmittel:** Um den pH-Wert der flüssigen Wasch- und Reinigungsmittel in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 7 Gew.% der Gesamtformulierung nicht. Der pH-Wert von flüssigen Wasch- und Reinigungsmitteln liegt bevorzugt zwischen 4 und 10 und bevorzugt zwischen 5.5 und 8.5. Der pH-Wert von flüssigen Weichspülern liegt bevorzugt zwischen 1 und 6 und bevorzugt zwischen 1.5 und 3.5.

**Farbstoffe:** Um den ästhetischen Eindruck der Textilbehandlungsmittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- und Reinigungsmittel und gegen Licht sowie keine ausgeprägte Substantivität gegenüber Textilfasern, um diese nicht anzufärben.

**Antiredepositionsmittel:** Geeignete Soil-Release-Polymere, die auch als "Antiredepositionsmittel" bezeichnet werden, sind beispielsweise nichtionische Celluloseether wie Methylcellulose und Methylhydroxypropylcellulose mit einem Anteil an Methoxygruppen von 15 bis 30 Gew.% und an Hydroxypropylgruppen von 1 bis 15 Gew.%, jeweils bezogen auf den nichtionischen Celluloseether sowie die aus dem Stand der Technik bekannten Polymere der Phthalsäure und/oder Terephthalsäure bzw. von deren Derivaten, insbesondere Polymere aus Ethylenterephthalaten und/oder Polyethylen- und/oder Polypropylenglykolterephthalaten oder anionisch und/oder nichtionisch modifizierten Derivaten von diesen. Geeignete Derivate umfassen die sulfonierten Derivate der Phthalsäure- und Terephthalsäure-Polymere.

**Optische Aufheller:** Optische Aufheller (sog. "Weisstöner") können den Wasch- und Reinigungsmitteln zugesetzt werden, um Vergrauungen und Vergilbungen der behandelten Textilen Flächengebilden zu beseitigen. Diese Stoffe ziehen auf die Faser auf und bewirken eine Aufhellung und vorgetäuschte Bleichwirkung, indem sie unsichtbare Ultraviolettstrahlung in sichtbares längenwelliges Lichtumwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiss ergibt. Geeignete Verbindungen stammen beispielsweiseaus den Substanzklassen der 4,4'-Diamino-2,2'-stilbendisulfonsaeuren (Flavonsäuren), 4,4'-Distyryl-biphenylen, Methylumbelliferone, Cumarine, Dihydrochinolinone, 1,3-Diarylpyrazoline, Naphthalsaeureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0 und 0.3 Gew.%, bezogen auf das fertige Wasch-und Reinigungsmittel, eingesetzt.

**Vergrauungsinhibitoren:** Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Naturgeeignet, beispielsweise Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, zum Beispiel abgebaute Stärke, Aldehydstärken usw. Auch Polyvinylpyrrolidon ist brauchbar. Bevorzugt werden jedoch Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxymethyl-cellulose und deren Gemische in Mengen von 0.1 bis 5 Gew.%, bezogen auf die Wasch- und Reinigungsmitteln, eingesetzt.

**Knitterschutzmittel:** Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können die Wasch- und Reinigungsmittel synthetische Knitterschutzmittel enthalten. Hierzu zählen beispielsweise synthetische Produkte auf der Basis von Fettsäuren, Fettsäureestern, Fettsäureamiden, -alkylolestern, - alkylolamiden oder Fettalkoholen, die meist mit Ethylenoxid umgesetzt sind, oder Produkte auf der Basis von Lecithin oder modifizierter Phosphorsäureester.

**Antimikrobielle Wirkstoffe:** Zur Bekämpfung von Mikroorganismen können die Wasch- und Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppensind beispielsweise Benzalkoniumchloride, Alkylarylsulfonate, Halogenphenole und Phenolmercuriacetat, wobei bei den erfindungemäßen Wasch- und Reinigungsmitteln auch gänzlich auf diese Verbindungen verzichtet werden kann.

**Konservierungsmittel:** Die erfindungsgemäßen Wasch- und Reinigungsmittel können Konservierungsmittel enthalten, wobei vorzugsweise nur solche eingesetzt werden, die kein oder nur ein geringes hautsensibilisierendes Potential besitzen. Beispiele sind Sorbinsäure und seine Salze, Benzoesäure und seine Salze, Salicylsäure und seine Salze, Phenoxyethanol, 3-lodo-2-propynylbutylcarbamat, Natrium N-(hydroxymethyl)glycinat, Biphenyl-2-ol sowie Mischungen davon. Ein geeignetes Konservierungsmittel stellt die lösungsmittelfreie, wässrige Kombination von Diazolidinylharnstoff, Natriumbenzoat und Kaliumsorbat (erhältlich als Euxyl^{®} K 500ex Schülke and Mayr) dar, welches in einem pH-Bereich bis 7 eingesetzt werden kann. Insbesondere eignen sich Konservierungsmittel auf Basis von organischen Säuren und/oder deren Salzen zur Konservierung der erfindungsgemäßen, hautfreundlichen Wasch- und Reinigungsmittel.

**Antioxidantien:** Um unerwünschte, durch Sauerstoffeinwirkung und andere oxidative Prozesse verursachte Veränderungen an den Wasch- und Reinigungsmitteln und/oder den behandelten textilen Flächengebilden zu verhindern, können die Wasch-und Reinigungsmittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite, Phosphonate und Vitamin E.

**Antistatika:** Ein erhöhter Tragekomfort kann aus der zusätzlichen Verwendung von Antistatika resultieren, die den Wasch- und Reinigungsmitteln zusätzlich beigefügt werden. Antistatika vergrößern die Oberflächenleitfähigkeit und ermöglichen damit ein verbessertes Abfließen gebildeter Ladungen. Antistatika sind in der Regel Substanzen mit wenigstens einem hydrophilen Molekuelliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsaeureester) und schwefelhaltige (Alkylsulfonate, Alkylsulfate) Antistatika unterteilen. Lauryl-(bzw. Stearyl-)dimethylbenzylammoniumchloride eignen sich als Antistatika für textile Flächengebilde bzw. als Zusatz zu Wasch- und Reinigungsmitteln, wobei zusätzlich ein Avivageeffekt erzielt wird.

**Schauminhibitoren:** Zur Verbesserung der Wiederbenetzbarkeit der behandelten textilen Flächengebilde und zur Erleichterung des Bügelns der behandelten textilen Flächengebilde können in den Textilbehandlungsmitteln beispielsweise Silikonderivate eingesetzt werden. Diese verbessern zusätzlich das Ausspülverhalten der Wasch- und Reinigungsmittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H-und/oder Si-Cl-Bindungen aufweisen. Die Viskositäten der bevorzugten Silikone liegen bei 25 °C im Bereich zwischen 100 und 100.000 mPas, wobei die Silikone in Mengen zwischen 0.2 und 5 Gew.-%, bezogen auf das gesamte Wasch- und Reinigungsmittel eingesetzt werden können.

**UV-Absorber:** Schließlich können die Wasch- und Reinigungsmittel auch UV-Absorber enthalten, die auf die behandelten textilen Flächengebilde aufziehen und die Lichtbeständigkeit der Fasern verbessern. Verbindungen, die diese gewünschten Eigenschaften aufweisen, sind beispielsweise die durch strahlungslose Deaktivierung wirksamen Verbindungen und Derivate des Benzophenons mit Substituenten in 2-und/oder4-Stellung. Weiterhin sind auch substituierte Benzotriazole, in 3-Stellung Phenyl-substituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

**Schwermetallkomplexbildner:** Um die durch Schwermetalle katalysierte Zersetzung bestimmter Waschmittel-Inhaltsstoffe zu vermeiden, können Stoffe eingesetzt werden, die Schwermetalle komplexieren. Geeignete Schwermetallkomplexbildner sind beispielsweise die Alkalisalze der Ethylendiamintetraessigsäure (EDTA) oder der Nitrilotriessigsäure (NTA) sowie Alkalimetallsalze von anionischen Polyelektrolyten wie Polymaleaten und Polysulfonaten. Eine bevorzugte Klasse von Komplexbildnern sind die Phosphonate, die in bevorzugten Textilbehandlungsmitteln in Mengen von 0.01 bis 2.5 Gew.-%, vorzugsweise 0.02 bis 2 Gew.% und insbesondere von 0.03 bis 1.5 Gew.% enthalten sind. Zu diesen bevorzugten Verbindungen zählen insbesondere Organophosphonate wie beispielsweise1-Hydroxyethan-1, 1-diphosphonsäure (HEDP), Aminotri(methylenphosphonsäure) (ATMP), Diethylentriaminpenta(methylen-phosphonsäure) (DTPMP bzw. DETPMP) sowie 2-Phosphonobutan-1,2,4-tricarbonsäure (PBS-AM), die zumeist in Form ihrer Ammonium- oder Alkalimetallsalze eingesetzt werden.

**Herstellung der Zubereitungen:** Die Herstellung fester WSR-Mittel kann nach den üblichen Verfahren erfolgen, wie beispielsweise Turmsprühverfahren, Wirbelschichttrockung, Agglomeration, Pulververmischung, Tablettierung, Granulierung, speziell SKET-Granulierung.

Die Herstellung flüssiger WSR-Mittel erfolgt ebenfalls mittels üblicher und bekannter Methoden und Verfahren, indem beispielsweise die Bestandteile einfach in Rührkesseln vermischt werden, wobei Wasser, nicht-wässrige Lösungsmittel und Tenside, zweckmäßigerweise vorgelegt werden und die weiteren Bestandteile portionsweise hinzugefügt werden. So können flüssige Wasch- und Reinigungsmittel hergestellt werden, indem die sauren Komponenten wie beispielsweise die linearen Alkylsulfonate, Zitronensäure, Borsäure, Phosphonsäure, die Fettalkoholethersulfate, usw. und die nichtionischen Tenside vorgelegt werden. Die Lösungsmittelkomponente wird vorzugsweise auch zu diesem Zeitpunkt hinzugegeben, die Zugabe kann aber auch zu einem späteren Zeitpunkt erfolgen. Zu diesen Komponenten kann das Verdickungsmittel, beispielsweise ein Polyacrylat gegeben werden. Anschließend wird eine Base wie beispielsweise NaOH, KOH, Triethanolamin oder Monoethanolamin gefolgt von der Fettsäure, falls vorhanden, hinzugefügt. Darauf folgend werden die restlichen Inhaltsstoffe und die restlichen Lösungsmittel des wässrigen flüssigen Wasch- und Reinigungsmittel zu der Mischung gegeben und der pH-Wert auf ungefähr 8,5 eingestellt. Abschließend können die zu dispergierenden Partikel zugegeben und durch Mischen homogen in dem wässrigen flüssigen Wasch- und Reinigungsmittel verteilt werden.

Die vorliegende Erfindung betrifft Parfümöle oder Mittel, die auf diese Weise parfümiert oder beduftet werden. Die Erfindung betrifft daher sowohl Pafümöle, kosmetischen Mittel, die Auftragungsmittel, als auch die Wasch- und Reinigungsmittel, einschließlich auch der parfümierten oder bedufteten Produkte, welche die Verbindungen der Formel (i), der Formel (ii) und/oder der Formel (iii) im Allgemeinen, oder das (E)-2-Methyl-but-2-endicarbonsäure-diethylester für sich, bzw., das Mesaconsäurediethylester in Mischung mit Citraconsäurediethylester sowie Itaconsäurediethylester im Besonderen, oder eine entsprechende Riechstoffmischung wie beschriebenen, wobei diese Parfümöle oder Mittel ausgewählt sind aus der Gruppe, die gebildet wird aus: Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreiniger, Geschirrspülmittel, Bad- und Sanitärreiniger, Scheuermilch, feste und flüssige WC-Reiniger, pulver- und schaumförmige Teppichreiniger, flüssigen Waschmittel, pulverförmige Waschmittel, Wäschevorbehandlungsmittel wie Bleichmittel, Einweichmittel und Fleckenentfernern, Wäscheweichspüler, Waschseife, Waschtabletten, Desinfektionsmittel, Oberflächendesinfektionsmittel sowie von Luftverbesserer in flüssiger, gelartiger oder auf einem festen Träger aufgebrachter Form, Aerosolsprays, Wachse und Polituren wie Möbelpolituren, Fußbodenwachse, Schuhcremes sowie Körperpflegemitteln wie z.B. feste und flüssige Seifen, Duschgele, Shampoos, Rasierseifen, Rasierschäumen, Badeöle, kosmetischen Emulsionen vom Öl-in-Wasser-, vom Wasser-in-Öl- und vom Wasser-in-Öl-in-Wasser-Typ wie z.B. Hautcremes und Hautlotionen, Gesichtscremes und -lotionen, Sonnenschutzcremes und -lotionen, Aftersun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukte wie z.B. Haarsprays, Haargelen, Haarlotionen, Haarspülungen, permanente und semipermanente Haarfärbemittel, Haarverformungsmittel wie Kaltwellen und Haarglättungsmittel, Haarwässer, Haarcremes und -lotionen, Deodorantien und Antiperspirantien wie z.B. Achselsprays, Roll-ons, Deosticks, Deocremes oder Produkte der dekorativen Kosmetik.

Insbesondere betrifft die Erfindung hierbei nach einem zehnten Aspekt Mittel die ausgewählt sind aus der Gruppe, die gebildet wird aus: festen und flüssigen Seifen, Duschgelen, Shampoos, Rasierseifen, Rasierschäumen, Badeölen, kosmetischen Emulsionen, Hautcremes und -lotionen, Gesichtscremes und Gesichtslotionen, Sonnenschutzcremes und -lotionen, Aftersuncremes und Aftersuncremeslotionen, Handcremes und -lotionen, Fußcremes und -lotionen, Enthaarungscremes und - lotionen, Aftershave-Cremes und -lotionen, Bräunungscremes und -lotionen, Haarsprays, Haargelen, Haarlotionen, Haarspülungen, permanenten und semipermanenten Haarfärbemitteln, Haarverformungsmitteln Haarwässern, Haarcremes und -lotionen, Deodorantien, Achselsprays, Roll-ons, Deosticks, Deocremes und Produkten der dekorativen Kosmetik sowie Parfüm-Extraits, Eau de Parfums, Eau de Toilettes, Rasierwässer, Eau de Colognes, Pre-shave-Produkte, Splash-Colognes, Erfrischungstüchern, sauren, alkalischen und neutralen Reinigungsmitteln, Fußbodenreinigern, Fensterglasreinigern, Geschirrspülmitteln, Bad- und Sanitär-reinigern, Scheuermilch, festen und flüssigen WC-Reinigern, pulver-und schaum-förmigen Teppichreinigern, flüssigen Waschmitteln, pulverförmigen Waschmitteln, Wäschevorbehandlungsmitteln, Bleichmitteln, Einweichmitteln und Flecken-entfernern, Wäscheweichspülern, Waschseifen, Waschtabletten, Desinfektions-mitteln, Oberflächendesinfektionsmitteln, Luftverbesserer in flüssiger, gelartiger oder auf einem festen Träger aufgebrachter Form, Aerosolsprays, Möbelpolituren, Fußbodenwachsen, Schuhcremes.

### HERSTELLVERFAHREN I

Ein elfter Gegenstand der vorliegenden Erfindung betrifft ein Verfahren zur Herstellung eines Mono- und/oder Diesters der Mesaconsäure umfassend die folgenden Schritte:
(i) Umsetzung von Itaconsäure mit Essigsäureanhydrid unter Erhalt von Itaconsäureanhydrid;
(ii) Isomerisierung von Itaconsäureanhydrid zu Citraconsäureanhydrid;
(iii) Veresterung von Citraconsäureanhydrid mit einem aliphatischen, araliphatischen oder aromatischen Alkohol mit 1 bis 10 Kohlenstoffatomen oder Diol mit 1 bis 5 Hydroxylgruppen unter Erhalt eines Mono- und/oder Diesters der Citraconsäure; und
(iv) Umlagerung des Mono- und/oder Diesters der Citraconsäure zu einem Mono- und/oder Diester der Mesaconsäure.

Das Verfahren wird beispielhaft anhand der bevorzugten Herstellung von (E)-2-Methyl-but-2-endicarbonsäure-diethylester (Mesaconsäurediethylester), bzw. ein Gemisch hieraus mit Citraconsäurediethylester und Itaconsäurediethylester im untenstehenden **Schema A** verdeutlicht.

Im ersten Reaktionsschritt wird im Allgemeinen eine Itaconsäure (bevorzugt Itaconsäurediethylester) mit einer in etwa äquimolaren Menge Acetanhydrid umgesetzt, wobei das cyclische Anhydrid gebildet und Essigsäure freigesetzt wird. Die Reaktion findet üblicherweise bei Temperaturen im Bereich von 50 bis 95 °C statt, anschließend wird die Essigsäure im Vakuum abdestilliert.

Im zweiten Schritt erfolgt die thermodynamisch gesteuerte Isomerisierung des Itaconsäureanhydrids zu Citraconsäureanhydrid. Die Reaktion wird vorzugsweise in einem Lösungsmittel durchgeführt und erfordert Temperaturen von 200 bis 250 °C. Auch hier wird das Lösungsmittel anschließend entfernt.

Es folgt im dritten Schritt die Veresterung mit Ethanol im Überschuss, die säurekatalysiert unter Rückfluss durchgeführt wird. Anschließend wird nicht umgesetzter Alkohol abdestilliert.

Die letzte vierte Stufe ist die Umlagerung des Citraconsäureesters (bevorzugt Citraconsäurediethylester) in die Mesaconsäure (bevorzugt den Diethylester der Mesaconsäure). Diese Reaktion findet vorzugsweise in einem geeigneten Lösungsmittel, wie beispielsweise MtBE bei Temperaturen im Bereich von 170 bis 200 °C statt; als Katalysator wird der Mischung Jod zugesetzt. Anschließend erfolgt wieder die destillative Abtrennung des Lösungsmittels.

Eine Illustration der Durchführung des Verfahrens von Stufe (i) bis Stufe (iv) findet sich im Beispielteil.

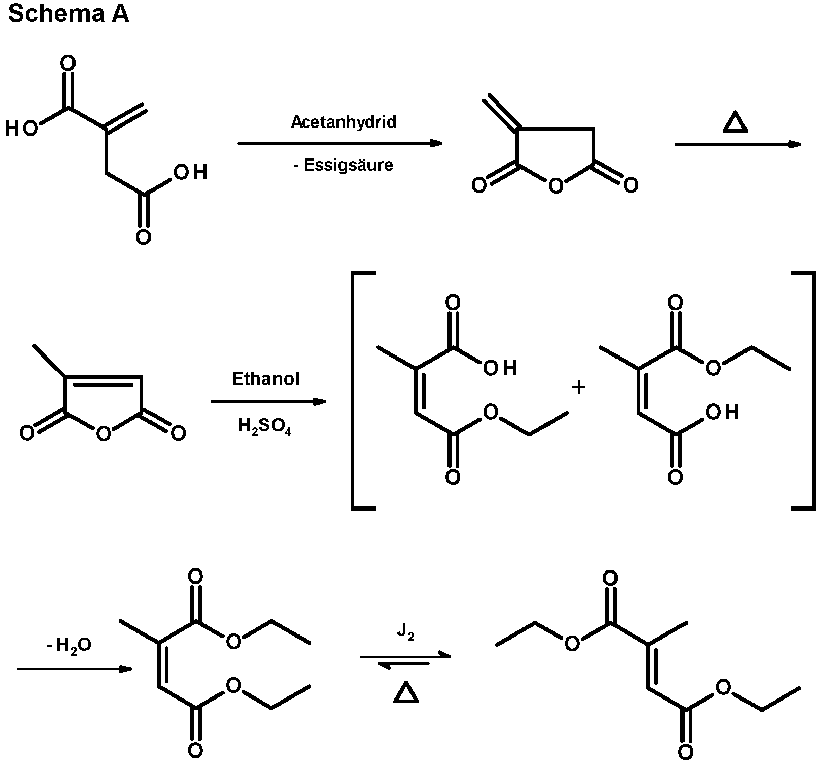

Besonders bevorzugt ist hierbei nach einer Weiterbildung der Erfindung das Verfahren zur Herstellung eines Mono- und/oder Diesters der Mesaconsäure, wobei in Schritt (ii) ein hoch siedendes Lösemittel verwendet wird, bevorzugt ein Polyalkylenglykol (PAG) mit der Struktur: RO-[CH2-C(CH3)HO]n -[CH2CH2O]m-H.

Das hochsiedende Lösungsmittel enthält vorzugsweise Lösungsmittel mit einem Siedepunkt von größer 250 °C, noch bevorzugter von größer 300 °C. Hier haben sich Polyalkylenglykol (PAG)-basierte Produkte bewährt. Solche sind z.B. Synalox^{®} 50-B SYNALOX ^{™} 50-xB, die als Schmierstoffe bekannt sind, und alkoholhaltige Materialien sind, die Oxyethylen und Oxypropylengruppen enthalten mit einer einzigen endständigen Hydroxylgruppe mit der Struktur:

RO-[CH₂-C(CH₃)HO]ₙ -[CH₂CH₂O]ₘ-H

Bekannt ist zu 50-50B SYNALOX: Molekulargewicht: 1300.

In einer zwölften Weiterbildung der Erfindung wird in Schritt (ii) ein Lösungsmittelsystem verwendet umfassend ein hoch siedendes Lösemittel, bevorzugt ein Polyalkylenglykol (PAG), mit einem Siedepunkt von größer 150 °C und weiterhin umfassend ein Co-Lösungsmittel mit einem Siedepunkt von zwischen 90 °C und 120 °C, bevorzugt Dioxan. Dieses System erhöht die Ausbeute deutlich, denn ohne das Schutzlösungsmittel neigt das destillierte Produkt im Mengenmaßstab mit größeren Reaktionsgefäßen auf dem längeren Destillationsweg zur Polymerisation. Somit konnte gezeigt werden, dass auch bei größeren Ansätzen die Ausbeute erhöht werden konnte, bzw. hoch bleibt, wenn das Schutzlösungsmittel einen Siedepunkt hat, sodass es ebenfalls mit dem Produkt mitdestilliert werden kann, um die Polymerisation des Produkts bei der Destillation zu verhindern. Besonders bevorzugt hat das Co-Lösungsmittel bzw. Schutzlösungsmittel einen Siedepunkt von zwischen 95 °C und 110°C, besonders bevorzugt von 100 °C bis 105 °C. Geeignet ist Dioxan.

Das Co-Lösungsmittel kann nach dem Destillationsschritt durch gängige Verfahren abgetrennt werden.

Weitere geeignete Co-Lösungsmittel sind:
- aliphatischen Kohlenwasserstoffe und spezieller die Paraffine, wie insbesondere Octan, Isooctan, Nonan, Decan, Undecan, Tetradecan; die aromatischen Kohlenwasserstoffe, wie insbesondere Toluen, die Xylene, Ethylbenzen, die Diethylbenzene, die Trimethylbenzene, Cumen, Pseudocumen, die Erdölfraktionen, die aus einem Gemisch von Alkylbenzenen bestehen, insbesondere die Fraktionen vom Typ Solvesso^{®},
- chlorierten aliphatischen Kohlenwasserstoffe, wie beispielsweise 1,1,2-Trichlorethan, Pentachlorethan, 1-lod-2-methylpropan, 1-Chlorhexan, 1-Chlor-2-ethylhexan; die chlorierten aromatischen Kohlenwasserstoffe und spezieller Chlorbenzen, die Chlortoluene,
- Ether und spezieller die aliphatischen Ether, wie Butylether, Isobutylether, Ethylhexylether, 1-Butoxy-2-methoxyethan, 1,1-Diethoxybutan, Amylether, Isoamylether, Dipropoxymethan; die aromatischen Ether, wie Phenylpropylether, Mesityloxid,
- nitrierten Verbindungen, wie Nitropropan, Nitrobenzen,
- aliphatischen, cycloaliphatischen oder aromatischen Ketone, vorzugsweise Methylethylketon, Methylisobutylketon, Diisobutylketon, Methyl-n-amylketon, Methylisoamylketon, Cyclohexanon, Methylcyclohexanon, Diacetonalkohol.

Die Co-Lösungsmittel können auch als eigenes Gemisch eingesetzt werden.

### HERSTELLVERFAHREN II

Ein dreizehnter Aspekt der vorliegenden Erfindung betrifft ein Verfahren zur Herstellung eines Mono- und/oder Diesters der Mesaconsäure umfassend die folgenden Schritte:
(i) Bereitstellen einer Lösung enthaltend mindestens einen Mono- und/oder Diesters der Citraconsäure und gegebenenfalls ein Lösungsmittel;
(ii) Versetzen der Lösung mit Jod;
(iii) Erhitzen der jodhaltigen Lösung auf etwa 170 bis etwa 200 °C und
(iv) gegebenenfalls destillative Aufreinigung des resultierenden Produktes.

Weiterhin ist bevorzugt, dass das Herstellungsverfahren II als Schritt (iv) im Herstellungsverfahren I eingesetzt wird. Der letzte Umlagerungsschritt wird somit nach dem Herstellungsverfahren II gleichsam im Anschluss an die Schritte (i) bis (iii) im Herstellungsverfahren I ausgeführt.

In einer vierzehnten Weiterbildung der vorliegenden Erfindung ist in den oben beschriebenen Verfahren I und II der Mono- und/oder Diester der Mesaconsäure ein (E)-2-Methyl-but-2-endicarbonsäureester, bevorzugt (E)-2-Methyl-but-2-endicarbonsäure-diethylester.

Ein bevorzugte Ausführungsform der vorliegenden Erfindung betrifft daher ein Verfahren zur Herstellung von (E)-2-Methyl-but-2-endicarbonsäure-diethylester umfassend die folgenden Schritte:
(i) Bereitstellen einer Lösung enthaltend mindestens Citraconsäurediethylester und gegebenenfalls ein Lösungsmittel;
(ii) Versetzen der Lösung mit Jod;
(iii) Erhitzen der jodhaltigen Lösung auf etwa 170 bis etwa 200 °C und
(iv) gegebenenfalls destillative Aufreinigung des resultierenden Produktes.

Vorzugsweise werden als Ausgangsstoffe Mono- und/oder Diester der Citraconsäure mit aliphatischen, araliphatischen oder aromatischen Alkoholen mit 1 bis 10 Kohlenstoffatomen oder Diolen mit 2 bis 6 Hydroxylgruppen eingesetzt. Insbesondere finden Mono- und Diester der Citraconsäure mit Methanol, Isopropylalkohol, den isomeren Butanolen, Ethylenglykol, Diethylenglykol, Propylenglykol, Dipropylenglykol oder Glycerin und insbesondere Ethanol Verwendung. Bezüglich der Besonderheiten des Verfahrens wird auf die Erläuterungen zum ersten Herstellverfahren, Stufe (iv), verwiesen, die hier mitgelten.

### Anwendungen

Ein fünfzehnter Aspekt der Erfindung betrifft ein Verfahren zum Vermitteln, Modifizieren oder Verstärken einer fruchtigen, birnenartigen Geruchsnote in einer Riechstoffmischung, einem Parfümöl, einem kosmetischen Mittel, einem Auftragungsmittel oder einem Wasch- und Reinigungsmittel, umfassend die folgenden Schritte:
(a1) Bereitstellen mindestens einer oder mehrerer erfindungsgemäßer Verbindung(en) der Formel (i), der Formel (ii) und/oder der Formel (iii) oder einer erfindungsgemäßen Riechstoffmischung, die diese Verbindungen(en) enthält und
(a2) Vermischen einer sensorisch wirksamen Menge dieses Stoffes oder dieser Stoffe mit einer Mischung weiterer Riechstoffe, die ausreicht, um in der fertigen Zubereitung eine fruchtige, birnenartige Geruchsnote hervorzurufen,
   oder
(b1) Bereitstellen mindestens einer oder mehrerer erfindungsgemäßer Verbindung(en) der Formel (i), der Formel (ii) und/oder der Formel (iii) oder einer erfindungsgemäßen Riechstoffmischung, die diese Verbindungen(en) enthält und
(b2) Vermischen einer sensorisch wirksamen Menge dieses Stoffes oder dieser Stoffe mit dem Parfümöl, kosmetischen Mittel, Auftragungsmittel oder dem Wasch- und Reinigungsmittel, die ausreicht, um in der fertigen Zubereitung eine fruchtige , birnenartige Geruchsnote hervorzurufen.

Bevorzugt sind hierbei die erfindungsgemäßen Verbindung(en) der Formel (i), der Formel (ii) und/oder der Formel (iii) jeweils die (E)-2-Methyl-but-2-endicarbonsäure-diethylester (Mesaconsäurediethylester), Citraconsäurediethylester und Itaconsäurediethylester, bzw. ein Gemisch von (E)-2-Methyl-but-2-endicarbonsäure-diethylester mit Citraconsäurediethylester und Itaconsäurediethylester.

Ein sechzehnter und siebzehnter Aspekt der Erfindung betrifft die Verwendung von Verbindungen der Formel (i), der Formel (ii) und/oder der Formel (iii) oder deren Mischungen im Allgemeinen, oder das *(E)*-2-Methyl-but-2-endicarbonsäure-diethylester für sich, bzw., Mesaconsäurediethylester in Mischung mit Citraconsäurediethylester sowie Itaconsäurediethylester im Besonderen, oder eine entsprechende Riechstoffmischung nach der Erfindung zum einen:
- als Riechstoffe, und zum anderen
- zum Vermitteln, Modifizieren oder Verstärken einer fruchtigen, birnenartigen Geruchsnote in einer Riechstoffmischung, einem Parfümöl, kosmetischen Mittel, einem Auftragungsmittel oder einem Wasch- und Reinigungsmittel.

Bezüglich bevorzugter Einsatzstoffe und Einsatzmengen wird auf die vorstehenden Ausführungen verwiesen, die hier mitgelten, so dass sich eine Wiederholung erübrigt. Als bevorzugte Verbindungen hierfür gelten die Verbindungen der Formel (i), der Formel (ii) und/oder der Formel (iii) oder deren Mischungen im Allgemeinen. Insbesondere wird noch einmal darauf hingewiesen, dass sich die jeweils besonders bevorzugten Varianten und Weiterbildungen jeglicher Ausgestaltungen der vorliegenden Erfindung jeweils auf Mesaconsäurediethylester, als Verbindung (i), bzw. eine Isomerenmischung, v.a. ein Gemisch von *(E)*-2-Methyl-but-2-endicarbonsäure-diethylester mit Citraconsäurediethylester und Itaconsäurediethylester bezieht, die diese Verbindung in einer Menge von wenigstens 50 Gew.-% enthält.

### BEISPIELE

### Beispiel für das Herstellverfahren 1: Darstellung von Mesaconsäurediethylester = (E)-2-Methyl-but-2-en-dicarbonsäure-diethylester

### 1. Stufe: Itaconsäureanhydrid

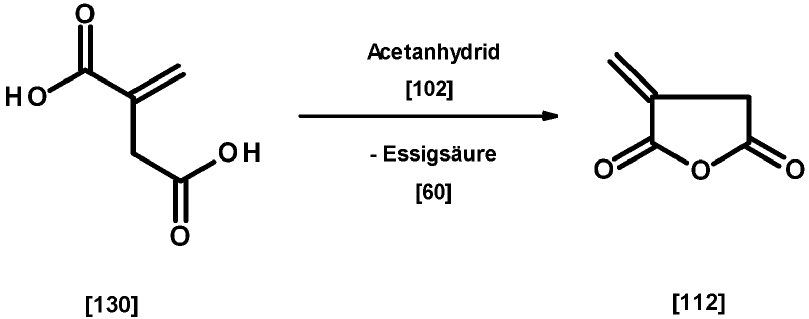

In einem 2-Liter-Dreihalskolben mit KPG-Rührer, 15-Vigreux-Kolonne und Kolonnenkopf wurden 612 g (6,0 mol) Acetanhydrid vorgelegt und unter Rühren mit 780 g (6,0 mol) fester Itaconsäure versetzt. Anschließend wurde langsam auf 80 °C erhitzt, bis der Feststoff gelöst war und noch 30 Minuten bei dieser Temperatur gerührt. Anschließend wurden bei einem Vakuum zwischen 150 und 10 mbar und einer Sumpftemperatur von 80 bis 82 °C ca. 680 g Essigsäure abdestilliert. Es verblieben ca. 690 g Sumpfprodukt, wobei die Rohausbeute 680 g betrug. Das Rohprodukt wurde direkt in Stufe 2 eingesetzt.

Ein 5 g Muster des Esters wurde per Kugelrohr Destillation aufgereinigt und gaschromatographisch analysiert und wies eine Zusammensetzung von 11 Gew.-% Citraconsäureanhydrid und 89 Gew.-% Itaconsäureanhydrid auf.

### 2. Stufe: Citraconsäureanhydrid

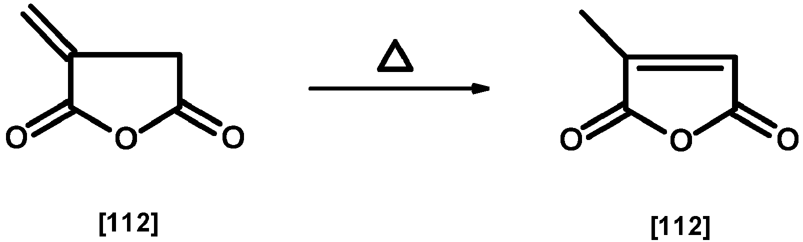

In einem 2-Liter-Dreihalskolben mit KPG-Rührer, 15-Vigreux-Kolonne und Kolonnenkopf wurden 100 g eines hoch siedenden Lösemittels (Synalox^{®} 50-B) vorgelegt und unter Rühren auf 230°C erhitzt. Anschließend wurden bei einem Vakuum von ca. 350 mbar 690 g Itaconsäureanhydrid (Rohprodukt Stufe 1), gelöst in 100 g Synalox innerhalb von 4 Stunden zu dosiert. Das Isomerisierungsprodukt Citraconsäureanhydrid wurde mit einem R/D-Verhältnis von 1:1 kontinuierlich abgenommen. Nach Beendigung der Dosierung wurde das Vakuum auf 10mbar angezogen und man erhielt insgesamt 630 g Destillat, was einer Ausbeute über beide Stufen von 94 % entsprach. Die GC-Reinheit betrug 99 %.

### 3. Stufe: Citraconsäurediethylester

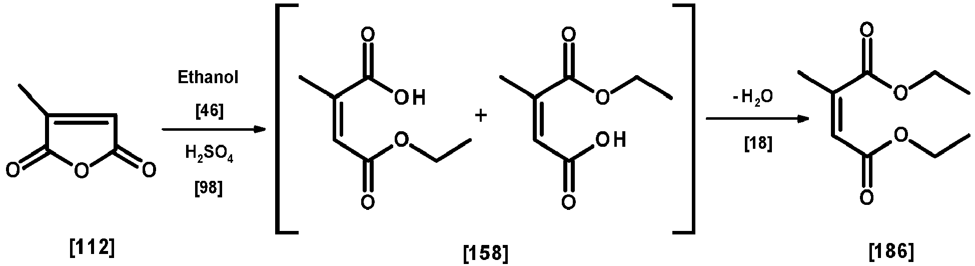

In einem 2-Liter-Dreihalskolben mit KPG-Rührer, 15-Vigreux-Kolonne und Kolonnenkopf wurden 780 g Ethanol vorgelegt und unter Rühren bei Raumtemperatur mit 630 g (5,63 mol) Citraconsäureanhydrid versetzt. Anschließend wurden innerhalb von 5 min 30 g konzentrierte Schwefelsäure zudosiert und 2 Stunden unter Rückfluss erhitzt. Danach wurden bei Normaldruck und einer Kopftemperatur von 86 °C 530 g eines Gemisches aus Ethanol/Wasser (80/20 Gew.-%) abdestilliert (R/D 1:3, Dauer ca. 80 min). Eine Probe wurde per Kugelrohr-Destillation aufgereinigt und gaschromatographisch analysiert, wobei 10 Gew.-% Citraconsäureanhydrid und 89 Gew.-% Citraconsäurediethylester gefunden wurden.

Anschließend wurden weitere 230 g Ethanol zugegeben und wiederum 1 Stunde unter Rückfluss bei einer Kopftemperatur von 84 °C erhitzt. Danach wurden 170 eines Gemisches aus Ethanol/Wasser (90/10 Gew.-%) abdestilliert (R/D 1:3, Dauer ca. 40 min). Eine Probe wurde per Kugelrohr-Destillation aufgearbeitet und gaschromatographisch analysiert, wobei 5 Gew.-% Citraconsäureanhydrid und 95 Gew.-% Citraconsäurediethylester gefunden wurde.

Erneut wurden 140 g Ethanol zugegeben, 1 Stunde unter Rückfluss erhitzt und bei einer Kopftemperatur von 82 °C ein Gemisch aus 110 g Ethanol/Wasser (90/10 Gew.-%) abdestilliert (R/D 1:3, Dauer ca. 25 min). Eine Probe wurde per Kugelrohr-Destillation aufgearbeitet und gaschromatographisch analysiert. Dabei wurden 2 Gew.-% Citraconsäureanhydrid und 96 Gew.-% Citraconsäurediethylester festgestellt.

Dann wurde auf Raumtemperatur abgekühlt und das Reaktionsprodukt nach Zugabe von 400 g MTBE mit 400 g Wasser gewaschen. Die organische Phase wurde anschließend zunächst unter starker Kohlendioxidentwicklung dreimal mit gesättigter Natriumbicarbonatlösung und danach mit 400 g einer 5 Gew.-%igen Kochsalzlösung gewaschen. Nach der Phasentrennung lagen insgesamt 1.970 g wässrige Phase (Abwasserfraktion I, pH 6), sowie 1.140 g organische Phase vor.

10% der organischen Phase wurden per Destillation aufgearbeitet und gaschromatographisch analysiert: Ausbeute: 74 g (71 % der Theorie); Reinheit: 99 % Citraconsäurediethylester.

### 4. Stufe: Mesaconsäurediethylester

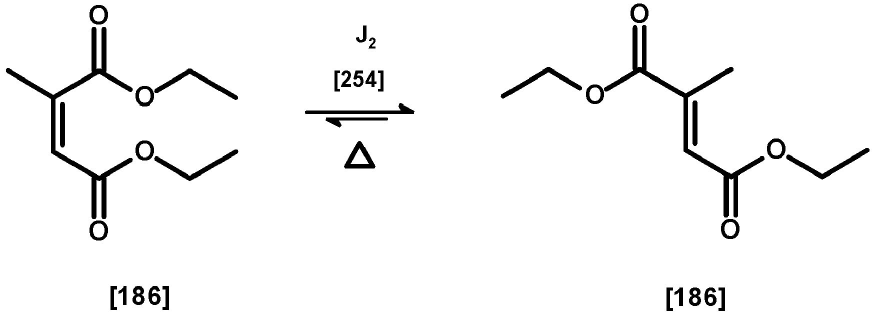

In einem 2-Ltr.-Dreihalskolben mit KPG-Rührer, 15-Vigreux-Kolonne und Kolonnenkopf wurden 1.100 g Citraconsäurediethylester in MTBE (Rohprodukt Stufe 3) vorgelegt und das Lösungsmittel bis zu einer Sumpftemperatur von 150 °C bei 50 bis 80 mbar abdestilliert. Man erhielt auf diese Weise etwa 320 g MTBE, das wieder eingesetzt werden konnte (siehe unten). Anschließend wurde auf ca. 80 °C abgekühlt, belüftet und mit 8 g (31 mmol) Jod unter Rühren versetzt. Es wurde 1 Stunde auf 190°C erhitzt. Eine Probe wurde per Kugelrohr-Destillation aufgereinigt und gaschromatographisch analysiert, dabei wurden 72,9 Gew.-% Mesaconsäurediethylester, 1,1 Gew.-% Itaconsäurediethylester und 25,9 Gew.-% Citraconsäurediethylester gefunden.

Da der Anteil des Mesaconsäureesters noch unter 80 Gew.-% lag, wurde eine weitere Stunde auf 190 °C erhitzt. Eine Probe wurde per Kugelrohr-Destillation aufgereinigt und gaschromatographisch analysiert, wobei nun 84,6 Gew.-% Mesaconsäurediethylester, 1,4 Gew.-% Itaconsäurediethylester und 13,8 Gew.-% Citraconsäurediethylester gefunden wurden.

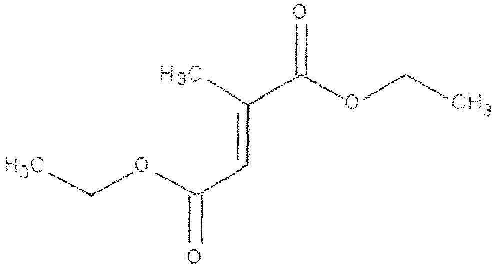

### Mesaconsäurediethylester = (E)-2-Methyl-but-2-en-dicarbonsäure-diethylester

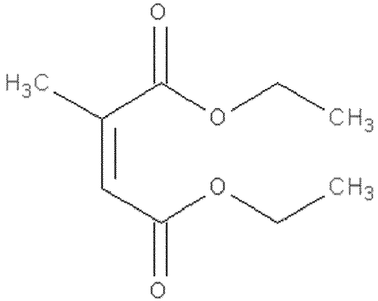

### Citraconsäurediethylester = (Z)-2-Methyl-but-2-ene-dicarbonsäure-diethylester

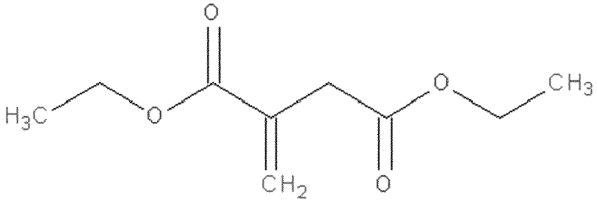

### Itaconsäurediethylester = 2-Methylen-Butandicarbonsäure-diethylester

Nach Abkühlung auf etwa 40 bis 45 °C wurde unter Rühren mit 320 g wiederverwendetem MTBE versetzt und vier Mal mit 300 g einer 40-Gew.-%igen Natriumhydrogensulfit-Lösung, einmal mit 300 g 5 Gew.-%iger Natriumbicarbonatlösung, sowie einmal mit 300 g 10 Gew.-%iger Kochsalzlösung gewaschen, wobei die Phasentrennungen innerhalb von 5 Minuten erfolgte. Man erhielt insgesamt etwa 1.800 g wässrige Phase (Abwasserfraktion II, pH 4) sowie etwa 1.000 g organische Phase. Die Reinigung des Produktes mit einer Einsatzmenge von etwa 1.000 g erfolgte über eine 10-cm-FKK-Kolonne entsprechend **Tabelle 1.**

**Tabelle 1: Destillative Reinigung**

| **Fraktion** | **T(Sumpf) [°C]** | **T(Kopf) [°C]** | **Druck [mbar]** | **R/D** | **Menge [g]** | **Analyse** |
|---|---|---|---|---|---|---|
| 1 | 76-96 | 55-69 | 300 | - | 250 | MTBE |
| 2 | 96-97 | 82 | 300 | - | 8 | Zwischenlauf |
| 3 | 97-115 | 82 | | - | 707 | Mesaconsäureester (84,6 %) |
| | | | | | 26 | Rückstand |
| | | | | | 29 | Kühlfalle |

Das Produkt färbte sich entweder schon während der Destillation rosa oder die Verfärbung stellte sich beim Stehenlassen ein. Zur Entfärbung wurde der Ester unter Rühren mit 14 g Aktivkohle versetzt, 30 Minuten bei Raumtemperatur gerührt und dann filtriert. Die Ausbeute betrug 705 g (95 % der Theorie).

### Umlagerungsschritt (ii) mit Lösungsmittelsystem

Im Folgenden wird ein verbessertes Verfahren unter Einsatz eines Lösungsmittelgemisches beschrieben.

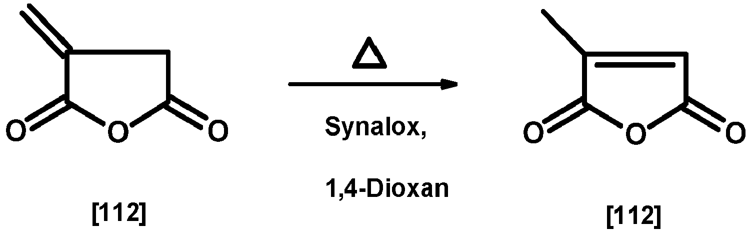

Bei der Herstellung von Citraconsäureanhydrid aus Itaconsäureanhydrid kommt das Lösungsmittelgemisch Synalox plus 1,4-Dioxan zum Einsatz. Diese bietet auch bei größeren Ansätzen eine gute Ausbeute. Hierzu wurde die folgende Durchführung angewandt.

Das Itaconsäureanhydrid wurde in Dioxan vorgelegt, bzw. als Rückstand aus dem Schritt (i) gewonnen (3 mol), und mit 390g 1,4-Dioxan versetzt und im Tropftrichter abgefüllt.
Hiernach erfolgt der Schritt (ii) gemäß der unten aufgeführten Vorschrift.

| **Durch- führung** | **Dauer [h:min]** | **Ges.- Rkt.-zeit [h:min]** | **Fr.** | **Temp. [°C]** | **Kopf [°C]** | **Dest. [g]** | **Be- merkungen** |
|---|---|---|---|---|---|---|---|
| 300g Synalox vorlegen und aufheizen | | 0:15 | | 0:15 | | 20-230 | |
| Dos. Itaconsäureanhydrid in Dioxan | 2:35 | 2:50 | 2 | 230-235 | 57-135 | 686 | Vakuum (350-20mbar) |

Das Produkt wird dann am Roti (60-70°C/100-20mbar) eingedampft und man erhält 296g (GC Purity = 96,6%) des Citraconsäureanhydrids mit einer Ausbeute von 85%.

### Herstellung weiterer Ester

In Analogie zu den oben genannten Verfahrensbeispielen zu (E)-2-Methyl-but-2-en-dicarbonsäurediethylester wurden weitere Ester hergestellt. Insbesondere wurden die Methyl-, Propyl- und n-Butyl-Ester der Mesaconsäure synthetisiert und analysiert. Hieraus ergaben sich insgesamt die vier Verbindungsvarianten:
- *(E)*-2-Methyl-but-2-endicarbonsäure-dimethylester,
- *(E)*-2-Methyl-but-2-endicarbonsäure-diethylester,
- *(E)*-2-Methyl-but-2-endicarbonsäure-dipropylester,
- *(E)*-2-Methyl-but-2-endicarbonsäure-dibutylester.

### Spektroskopische Daten:

### (E)-2-Methyl-but-2-endicarbonsäure-dimethylester

¹H-NMR (400 MHz, CDCl₃): δ = 2.31 (d, J = 1.6 Hz, 3H), 3.78 (s, 3H), 3.28 (s, 3H), 6.80 (q, J = 1.6 Hz, 1H).

¹³C NMR (CDCl₃, 101 MHz): δ = 167.6 (C=O), 166.3 (C=O), 143.7 (C), 126.5 (CH), 52.6 (CH₃), 51.7 (CH₃), 14.3 (CH₃).

MS (70 eV): 158, 143, 127, 113, 99.

### (E)-2-Methyl-but-2-en-dicarbonsäurediethylester

¹H-NMR (400 MHz, CDCl₃): δ = 1.32 ppm *(t, J =* 7.1 Hz, 3H), 1.33 ppm *(t, J =* 7.1 Hz, 3H), 2.29 (d, J = 1.6 Hz, 3H), 4.23 (q, *J* = 7.1 Hz, 2H), 4.26 (q, *J* = 7.1 Hz, 2H), 6.78 (q, 1,6 Hz, 1H).

¹³C NMR (CDCl₃, 101 MHz): δ = 167.16 (C=O), 165.97 (C=O), 143.76 (C), 126.66 (CH), 61,57 (CH₂), 60,62 (CH₂), 14.27 (CH₃), 14.20 (CH₃), 14.13 (CH₃).

MS (70 eV): 186, 157, 141, 113, 99.

### (E)-2-Methyl-but-2-endicarbonsäure-dipropylester

¹H-NMR (400 MHz, CDCl₃): δ = 0.98 *(t, J* = 7.4 Hz, 3H), 0.98 *(t, J* = 7.4 Hz, 3H), 1.79-1.64 (m, 4H), 2.29 (d, *J* = 1.6 Hz, 3H), 4.13 (t, *J* = 6.5 Hz, 2H), 4.16 *(t, J* = 6.5 Hz, 2H), 6.79 (q, *J* = 1.6 Hz, 1H).

¹³C NMR (CDCl₃, 100 MHz): δ = 167.3 (C), 166.1 (C), 143.7 (C), 126.7 (CH), 67.2 (CH₂), 66.3 (CH₂), 21.9 (CH₂), 21.6 (CH₂), 14.3 (CH₃), 10.4 (CH₃), 10.4 (CH₃).

MS (70 eV): 214, 199 185, 155, 131, 96.

### (E)-2-Methyl-but-2-endicarbonsäure-dibutylester

¹H-NMR (400 MHz, CDCl₃): δ = 0.95 *(t, J* = 7.3 Hz, 3H), 0.96 *(t, J* = 7.3 Hz, 3H), 1.47-1.36 (m, 4H), 1.72-1.61 (m, 4H), 2.29 (d, *J* = 1.6 Hz, 3H), 4.18 (t, *J =* 6.7 Hz, 2H), 4.20 *(t, J* = 6.6 Hz, 2H), 6.77 (q, *J = 1.6 Hz,* 1H).

¹³C NMR (CDCl₃, 100 MHz): δ = 167.3 (C), 166.1 (C), 143.7 (C), 126.7 (CH), 65.5 (CH₂), 64.6 (CH₂), 30.5 (CH₂), 30.6 (CH₂), 19.19 (CH₂), 19.18 (CH₂), 14.3 (CH₃), 13.7 (CH₃).

MS (70 eV): 242, 227, 213, 169, 131, 68.

Die folgende Tabelle 2 zeigt die Ergebnisse des GC Profils und des Geruchstests für die hergestellten Ester. Die Abkürzungen DD und TN stehen jeweils für die fachmännischen Begriffe "dry down" und "top note".

**Tabelle 2**

| **Verbindungsstruktur** | **GC Profil: DB Wachs** | **Duftnote:** |
|---|---|---|
| Methyl | | |
| ***(E)*-2-Methyl-but-2-endicarbonsäure-**dimethylester | 86% | DD: Nichts |
| *(Z)*-2-Methyl-but-2-endicarbonsäure-dimethylester | 11% | |
| | | TN: anis, anethol, fruchtig, ananas, grün. |
| 2-Methylenbutandicarbonsäuredimethylester | 3% | |

| Ethyl | | |
|---|---|---|
| *(E)*-2-Methyl-but-2-en-dicarbonsäure-diethylester | 85% | DD: butterig, zuckerig |
| *(Z)*-2-Methyl-but-2-en-dicarbonsäure-diethylester | 13.8% | |
| | | TN: fruchtig, grün, Birne |
| 2-Methylenbutandicarbonsäurediethylester | 1.4% | |

| Propyl | | |
|---|---|---|
| *(E)*-2-Methyl-but-2-endicarbonsäure-dipropylester | 82% | DD: gleich wie Ethyl; |
| *(Z)*-2-Methyl-but-2-endicarbonsäure-dipropylester | 9% | |
| 2-Methylenbutandicarbonsäuredipropylester | 6% | TN: fruchtig, zuckerig, linear, veilchen, |

| n-Butyl | | |
|---|---|---|
| *(E)*-2-Methyl-but-2-endicarbonsäure-dibutylester | 88% | DD: gleich wie Ethyl; |
| *(Z)*-2-Methyl-but-2-endicarbonsäure-dibutylester | 3% | |
| | | TN: grüner als anderen, aber schwach. grün, fruchtig |
| 2-Methylenbutandicarbonsäuredibutylester | 8% | |

### ANWENDUNGSTECHNISCHE BEISPIELE

### BEISPIEL 1: Shower Gel

| **Material** | **Hersteller** | **INCI-Name** | **Gew.-%** |
|---|---|---|---|
| Entionisiertes Wasser | | Wasser | 76,3 |
| Plantacare PS 10 | Cognis Deutschland GmbH | Sodium Laureth Sulfate, Lauryl Glucoside | 20,0 |
| Dragocid Liquid | Symrise | Phenoxyethanol, Methylparaben, Ethylparaben, Butylparaben, Propylparaben, Isobutylparaben | 0,5 |
| Natrium Chlorid | | Sodium Chloride | 1,4 |
| Citronensäure Monohydrat kristallin | | Citric Acid | 1,3 |
| Parfumöl A1 oder A2 aus diesem Beispiel 1 | Symrise | Parfum (Fragrance) | 0,5 |

### Parfümkomposition für ein Shower Gel

| | **A1 Vergleichs beispiel** | **A2 Erfindungs -gemäßes Beispiel** |
|---|---|---|
| HEXENAL TRANS-2 10% DPG | 5 | 5 |
| HEXENOL BETA GAMMA CIS,TRANS-3 | 1 | 1 |
| HEXENOL TRANS-2 10% DPG | 1 | 1 |
| HEXENYLACETAT CIS,TRANS-3 10% DPG | 6 | 6 |
| HEXENYLISOBUTYRAT CIS-3 10% DPG | 5 | 5 |
| VERTOCITRAL 10% DPG | 7 | 7 |
| STYROLYLACETAT 10% DPG | 5 | 5 |
| CITRONENOEL ITAL. | 10 | 10 |
| BUTYLACETAT | 10 | 10 |
| HEXYLACETAT | 30 | 30 |
| ISOAMYLACETAT | 11 | 11 |
| JASMAPRUNAT | 40 | 40 |
| PRENYLACETAT | 3 | 3 |
| ALDEHYD C14 SOG | 4 | 4 |
| PYROPRUNAT | 10 | 10 |
| FRAMBINON^{®} 10% DPG | 5 | 5 |
| MALTOL 1% DPG | 2 | 2 |
| DIMETHYLBENZYLCARBINYLBUTYRAT | 15 | 15 |
| GERANYLACETAT PUR | 220 | 220 |
| DAMASCENON | 2 | 2 |
| ISODAMASCON^{®} | 5 | 5 |
| HEDION | 100 | 100 |
| AGRUMEX HC | 14 | 14 |
| AMBRETTOLIDE | 1 | 1 |
| ETHYLENBRASSYLAT | 3 | 3 |
| GLOBALIDE^{®} | 5 | 5 |
| **MESACONSÄUREDIETHYLESTER** | -- | 80 |
| DIPROPYLENGLYCOL | 480 | 400 |
| | 1000 | 1000 |

Mesaconsäurediethylester lässt sich einfach und stabil in beliebige Parfümkompositionen einarbeiten und bringt eine natürliche, fruchtige und strahlende Note ein.

### BEISPIEL 2: Bodylotion

| **Komponente** | **Gew.-%** | **Gew.-%** |
|---|---|---|
| Paraffinöl | 5,00 | 5,00 |
| Isopropylpalmitat | 5,00 | 5,00 |
| Cetylalkohol | 2,00 | 2,00 |
| Bienenwachs | 2,00 | 2,00 |
| Ceteareth-20 | 2,00 | 2,00 |
| PEG-20-Glycerylstearat | 1,50 | 1,50 |
| Glycerin | 3,00 | 3,00 |
| Phenoxyethanol | 0,50 | - |
| Parabene (Mischung aus Methyl-, Ethyl-, Propyl-, Butyl-, Isobutylparaben) | - | 0,50 |
| Parfümöl B1 oder B2 | 0,50 | 0,50 |
| Wasser | Ad 100 | Ad 100 |

### Parfümöle für diese Bodylotion

| **Riechstoffe** | **Parfümöl B1** Vergleich | **Parfümöl B2** erfindungsgemäß |
|---|---|---|
| NONADIENAL TRANS,CIS-2,6 5% TEC 20% DPG | 2,0 | 2,0 |
| ETHYL ACETOACETATE | 3,0 | 3,0 |
| FARENAL^{®} 10% DPG | 5,0 | 5,0 |
| VERTOCITRAL | 3,0 | 3,0 |
| CYCLOGALBANAT^{®} 10% DPG | 2,0 | 2,0 |
| STYRALYL ACETATE | 3,0 | 3,0 |
| MELONAL^{®} | 0,5 | 0,5 |
| DIHYDRO MYRCENOL | 15,0 | 15,0 |
| LINALYL ACETATE | 20,0 | 20,0 |
| LEMON OIL TERPENES FLAVOR WONF | 8,0 | 8,0 |
| EUCALYPTOL NAT. 10% DPG | 0,5 | 0,5 |
| HEXYL ACETATE | 1,5 | 1,5 |
| ISOAMYL ACETATE 10% DPG | 4,0 | 4,0 |
| PRENYL ACETATE 10% DPG | 4,0 | 4,0 |
| ALDEHYDE C14 SO-CALLED | 2,0 | 2,0 |
| ETHYL METHYL BUTYRATE-2 | 1,0 | 1,0 |
| ALLYL CYCLOHEXYL PROPIONATE | 2,0 | 2,0 |
| ALDEHYDE C16 SO-CALLED | 1,0 | 1,0 |
| FRAGOLANE^{®} | 0,5 | 0,5 |
| MAJANTOL^{®} | 25,0 | 25,0 |
| LINALOOL | 40,0 | 40,0 |
| DIMETHYL BENZYL CARBINOL | 10,0 | 10,0 |
| TERPINEOL PURE | 10,0 | 10,0 |
| PHENIRAT^{®} | 30,0 | 30,0 |
| CITRONELLOL 950 | 15,0 | 15,0 |
| GERANIOL 60 | 10,0 | 10,0 |
| CITRONELLYL ACETATE EXTRA | 2,0 | 2,0 |
| HEDIONE | 90,0 | 90,0 |
| HEXYL CINNAMIC ALDEHYDE ALPHA | 35,0 | 35,0 |
| HEXYL SALICYLATE | 160,0 | 160,0 |
| METHYL OCTIN CARBONATE 10% DPG | 2,0 | 2,0 |
| CALONE 1951 10% DPG | 1,0 | 1,0 |
| GALAXOLIDE 50% IN IPM | 20,0 | 20,0 |
| ANETHOL SUPRA 21,5 CELSIUS | 2,0 | 2,0 |
| AGRUMEX HC | 15,0 | 15,0 |
| ORYCLON SPECIAL | 40,0 | 40,0 |
| **MESACONSÄUREDIETHYLESTER** | - | **15,0** |
| DIPROPYLENE GLYCOL | 415,0 | 400,0 |
| | 1.000,0 | 1.000,0 |

Durch Zusatz von Mesaconsäurediethylester (MSDEE) zur Duftzusammensetzung ergibt sich eine frischere, fruchtige Note. Außerdem wird die Topnote strahlendund der Nachgeruch pflegender.

### BEISPIEL 3: Weichspüler

| **Material** | **Hersteller** | **Chemischer Name** | **Funktion** | **Gew.- %** |
|---|---|---|---|---|
| Entionisiertes Wasser | | Wasser | Lösungsmittel | 72,4 |
| Rewoquat WE 18 | Evonic Goldschmidt GmbH | Dialkylesterammoniumethosulfate | Kationisches Tensid | 16,6 |
| Mergal K9N | Honeywell Austria GmbH | 5-Chloro-2-methyl-3-(2H)-isothiazolone und 2-methyl-3-(2H)-isothiazolone | Konservierungsmittel | 0,10 |
| Dow Corning 1520 Antifoam | Dow Corning GmbH, Deutschland | Polydimethyl-siloxane | Entschäumer | 0,30 |
| Magnesium Chlorid 1%ige Lösung | | Magnesium Chlorid Lösung | Konsistenzgeber | 10,00 |
| Parfumöl C1 oder C2 | Symrise | | Parfum (Fragrance) | 0,60 |

### Parfümöle für diesen Weichspüler

| **Riechstoffe** | **Parfümöl C1** Vergleich | **Parfümöl C2** erfindungsgemäß |
|---|---|---|
| ALDEHYDE C10 | 0,5 | 0,5 |
| ALDEHYDE C11 ISO | 2,0 | 2,0 |
| ALDEHYDE C11 UNDECYLENIC | 12,0 | 12,0 |
| ALDEHYDE C12 MNA | 6,0 | 6,0 |
| FARENAL^{®} | 1,0 | 1,0 |
| VERTOCITRAL | 8,0 | 8,0 |
| ALLYL AMYL GLYCOLATE | 1,0 | 1,0 |
| STYRALYL ACETATE | 1,5 | 1,5 |
| DIHYDRO MYRCENOL | 65,0 | 65,0 |
| AGRUNITRIL | 1,0 | 1,0 |
| PEONILE | 15,0 | 15,0 |
| METHYL ANTHRANILATE | 10,0 | 10,0 |
| NEROLIONE 10% DPG | 1,5 | 1,5 |
| ROSEMARY OIL BM | 7,0 | 7,0 |
| SAGE OFFICINALE OIL DALM. | 3,0 | 3,0 |
| ISOBORNYL ACETATE | 40,0 | 40,0 |
| PRENYL ACETATE | 0,5 | 0,5 |
| ISOAMYL BUTYRATE | 0,5 | 0,5 |
| ALDEHYDE C14 SO-CALLED | 5,0 | 5,0 |
| MANZANATE | 0,5 | 0,5 |
| MUGETANOL | 5,0 | 5,0 |
| DIMETHYL BENZYL CARBINYL ACETATE | 2,5 | 2,5 |
| ROSE OXIDE D | 3,0 | 3,0 |
| ANTHER | 0,5 | 0,5 |
| PHENYLETHYL ALCOHOL | 35,0 | 35,0 |
| CITRONELLOL 950 | 15,0 | 15,0 |
| GERANIOL 60 | 10,0 | 10,0 |
| ISODAMASCON^{®} | 2,0 | 2,0 |
| ROSACETATE | 35,0 | 35,0 |
| CRESYL METHYL ETHER PARA | 0,5 | 0,5 |
| BENZYL ACETATE | 20,0 | 20,0 |
| HEXYL CINNAMIC ALDEHYDE ALPHA | 40,0 | 40,0 |
| METHYL BENZOATE | 1,0 | 1,0 |
| AMYL SALICYLATE N/ISO | 10,0 | 10,0 |
| BENZYL SALICYLATE | 70,0 | 70,0 |
| MYSORE ACETATE | 15,0 | 15,0 |
| PARMANYL^{®} | 0,5 | 0,5 |
| EUGENOL | 10,0 | 10,0 |
| ETHYL VANILLIN | 0,5 | 0,5 |
| COUMARIN | 3,0 | 3,0 |
| AGRUMEX HC | 45,0 | 45,0 |
| HERBAFLORAT | 25,0 | 25,0 |
| AMBERWOOD^{®} F | 5,0 | 5,0 |
| ISO E SUPER | 170,0 | 170,0 |
| PATCHOULI OIL DECOL. | 2,5 | 2,5 |
| SANDRANOL^{®} | 10,0 | 10,0 |
| ISOBUTYL QUINOLINE | 1,0 | 1,0 |
| EVERNYL | 0,5 | 0,5 |
| AMBROXIDE | 1,0 | 1,0 |
| GALAXOLIDE 50% IN IPM | 120,0 | 120,0 |
| INDOFLORO CRYST. | 0,5 | 0,5 |
| **MESACONSÄUREDIETHYLESTER** | - | **60,0** |
| DIPROPYLENE GLYCOL | 220,0 | 160,0 |
| | 1.000,0 | 1.000,0 |

Durch Zusatz von Mesaconsäurediethylester (MSDEE) zur Duftzusammensetzung ergibt sich eine natürlichere, runde, transparente und fruchtige Note.

### BEISPIEL 4: Seife

| **Material** | **Hersteller** | **Chemischer Name** | **Funktion** | **Gew.-%** |
|---|---|---|---|---|
| Entionisiertes Wasser | | Water | Lösungsmittel | 2,0 |
| Seifen Basen Mix | Verschiedene | Sodium tallowates / palmitates | Tenside | 95,8 |
| Titan Dioxid | Kronos Titan GmbH, Deutschland | Titanium Dioxide | Farbstoff / Aufheller | 1,0 |
| Parfümöl D1 oder D2 | Symrise | | Parfum (Fragrance) | 1,2 |

### Parfümöle für diese Seife

| **Riechstoffe** | **Parfümöl D1** (Vergleich) | **Parfümöl D2** (erfindungsgemäß) |
|---|---|---|
| ALDEHYDE C 8 | 10,0 | 10,0 |
| ALDEHYDE C10 | 14,0 | 14,0 |
| ALDEHYDE C11 UNDECYLIC | 5,0 | 5,0 |
| ALDEHYDE C12 MNA | 4,0 | 4,0 |
| ISOAMYL ALCOHOL | 6,0 | 6,0 |
| VERTOCITRAL | 4,0 | 4,0 |
| ALLYL AMYL GLYCOLATE | 4,0 | 4,0 |
| DIHYDRO MYRCENOL | 120,0 | 120,0 |
| CITRAL 95 | 30,0 | 30,0 |
| AGRUNITRIL | 80,0 | 80,0 |
| CITRONITRILE | 40,0 | 40,0 |
| CITRYLAL | 4,0 | 4,0 |
| ORANGE OIL TERPENES | 250,0 | 250,0 |
| METHYL ANTHRANILATE | 1,5 | 1,5 |
| TERPINEOL HEAD FRACTION | 10,0 | 10,0 |
| EUCALYPTOL NAT. | 10,0 | 10,0 |
| THYMOL CRYST | 6,0 | 6,0 |
| BORNYL ACETATE L CRYST. | 82,5 | 82,5 |
| CAMPHOR DL | 20,0 | 20,0 |
| JASMAPRUNAT | 5,0 | 5,0 |
| ALDEHYDE C14 SO-CALLED | 2,0 | 2,0 |
| MANZANATE | 0,5 | 0,5 |
| ALLYL HEPTOATE | 2,5 | 2,5 |
| ROSE OXIDE L | 0,5 | 0,5 |
| DAMASCONE ALPHA | 0,5 | 0,5 |
| HEDIONE | 10,0 | 10,0 |
| AMYL SALICYLATE N/ISO | 60,0 | 60,0 |
| HEXYL SALICYLATE | 35,0 | 35,0 |
| COUMARIN | 2,5 | 2,5 |
| AGRUMEX HC | 30,0 | 30,0 |
| ORYCLON SPECIAL | 55,0 | 55,0 |
| PATCHOULI OIL DECOL. | 2,0 | 2,0 |
| SANDRANOL^{®} | 1,5 | 1,5 |
| TONALIDE | 2,0 | 2,0 |
| **MESACONSÄUREDIETHYLESTER** | - | **40,0** |
| DIPROPYLENE GLYCOL | 130,0 | 90,0 |
| | 1.000,0 | 1.000,0 |

Der Zusatz von Mesaconsäurediethylester (MSDEE) ergibt natürlichere, transparente, birnenartige und fruchtige Noten. Weiterhin verleiht Mesaconsäurediethylester der Komposition mehr Kraft und Fülle.

### BEISPIEL 5: Perlglanz-Shampoo

| **Material** | **Hersteller** | **INCI-Name** | **Gew.-%** |
|---|---|---|---|
| Entionisiertes Wasser | | Water | 71,5 |
| Plantacare PS 10 | BASF Personal Care & Nutrition GmbH | Sodium Laureth Sulfate, Lauryl Glucoside | 20,0 |
| Euperlan PK 771 | BASF Personal Care & Nutrition GmbH | Glycol Distearate, Sodium Lauryl Sulfate, Cocamide MEA, Laureth-10 | 6,0 |
| Dragocid Liquid | Symrise AG | Phenoxyethanol, Methylparaben, Ethylparaben, Butylparaben, Propylparaben, Isobutylparaben | 0,5 |
| Natriumchlorid | | Sodium Chloride | 1,4 |
| Zitronensäure Monohydrat kristallin | | Citric Acid | 0,1 |
| Parfümöl G1 oder G2 | Symrise AG | Parfum (Fragrance) | 0,5 |

### Parfümöle für dieses Perlglanz-Shampoo:

| **Riechstoffe** | **Parfümöl G1** (Vergleich) | **Parfümöl G2** (erfindungsgemäß) |
|---|---|---|
| FARENAL^{®} | 3,0 | 3,0 |
| FLORAZON | 0,5 | 0,5 |
| HEXENYL ACETATE CIS-3 | 3,0 | 3,0 |
| VERTOCITRAL | 1,0 | 1,0 |
| DYNASCONE 10% DPG | 2,0 | 2,0 |
| CYCLOGALBANAT^{®} | 2,0 | 2,0 |
| STYRALYL ACETATE | 1,5 | 1,5 |
| DIHYDRO MYRCENOL | 6,0 | 6,0 |
| OXANTHIA 50% IN TEC 10% DPG | 10,0 | 10,0 |
| LEMON OIL ITAL. | 10,0 | 10,0 |
| ORANGE OIL BRASIL | 50,0 | 50,0 |
| HEXYL ACETATE | 2,0 | 2,0 |
| ISOAMYL ACETATE | 0,5 | 0,5 |
| PRENYL ACETATE | 0,5 | 0,5 |
| ETHYL BUTYRATE 10% DPG | 2,0 | 2,0 |
| ALDEHYDE C14 SO-CALLED | 25,0 | 25,0 |
| DECALACTONE GAMMA | 5,0 | 5,0 |
| ETHYL METHYL BUTYRATE-2 | 1,0 | 1,0 |
| ALLYL CAPROATE | 1,5 | 1,5 |
| ALLYL CYCLOHEXYL PROPIONATE | 3,0 | 3,0 |
| ALLYL HEPTOATE | 2,5 | 2,5 |
| MELOZONE | 2,0 | 2,0 |
| CALONE 1951 | 0,5 | 0,5 |
| MUGETANOL | 10,0 | 10,0 |
| LINALOOL | 25,0 | 25,0 |
| DIMETHYL BENZYL CARBINYL ACETATE | 6,0 | 6,0 |
| DIMETHYL BENZYL CARBINYL BUTYRAT | 4,0 | 4,0 |
| PHENYLETHYL ACETATE | 1,5 | 1,5 |
| PHENYLETHYL ALCOHOL | 15,0 | 15,0 |
| CITRONELLOL 950 | 10,0 | 10,0 |
| GERANIOL SUPER | 5,0 | 5,0 |
| GERANYL ACETATE PURE | 15,0 | 15,0 |
| ISODAMASCON^{®} | 2,0 | 2,0 |
| BENZYL ACETATE | 15,0 | 15,0 |
| HEDIONE | 90,0 | 90,0 |
| HEXYL CINNAMIC ALDEHYDE ALPHA | 35,0 | 35,0 |
| JASMONE CIS | 0,5 | 0,5 |
| BENZYL SALICYLATE | 80,0 | 80,0 |
| HEXENYL SALICYLATE CIS-3 | 30,0 | 30,0 |
| ISORALDEINE 70 | 35,0 | 35,0 |
| HERBAFLORAT | 15,0 | 15,0 |
| ISO E SUPER | 15,0 | 15,0 |
| ISOBORNYL CYCLOHEXANOL | 30,0 | 30,0 |
| BRAHMANOL^{®} | 10,0 | 10,0 |
| AMBROXIDE | 1,5 | 1,5 |
| GLOBALIDE^{®} | 5,0 | 5,0 |
| GALAXOLIDE 50% IN DPG | 120,0 | 120,0 |
| **MESACONSÄUREDIETHYLESTER** | - | **65,0** |
| DIPROPYLENE GLYCOL | 290,0 | 225,0 |
| | 1.000,0 | 1.000,0 |

Bei einer Dosierung von 0.5% des Parfümöls im Shampoo ergibt sich folgender Befund: der Zusatz Mesaconsäurediethylester (MSDEE) in Parfümöl G2 verstärkt den strahlend-fruchtigen Charakter. Zudem wird die Diffusivität erhöht.

### BEISPIEL 6: Transparente Deo-Stifte (Formulierung A, B) bzw. Deo-Creme-Stifte (Formulierung C, D)

| **Komponente (in Gew.-%)** | **A** | **B** | **C** | **D** |
|---|---|---|---|---|
| Aluminium Zirconium Tetrachlorohydrat - Glycin Komplex | 25,00 | 20,00 | 25,00 | 20,00 |
| Dimethicon (10 Cst) | - | - | 5,00 | 5,00 |
| Cyclopentasiloxan | - | 0,50 | 1,00 | 0,50 |
| Petrolatum | 5,00 | 4,70 | 5,00 | 5,00 |
| Ozokerit | 1,00 | 1,50 | - | - |
| Stearylalkohol | 12,00 | 12,00 | - | - |
| 2-Butyloctansäure | 0,50 | - | 0,50 | - |
| Wachs | - | - | 1,25 | 1,25 |
| PPG-14 Butylether | 9,00 | 9,00 | - | - |
| Gehärtetes Rapsöl | - | - | 5,00 | 5,00 |
| Siliciumdioxid | - | - | 1,00 | - |
| Farnesol | 0,25 | - | 0,25 | - |
| Paraffinöl | 0,50 | 0,50 | - | - |
| Hydriertes Rizinusöl (castor wax) | 3,50 | 3,50 | - | - |
| Talk | 4,00 | 4,00 | - | - |
| Behenylalkohol | 0,20 | 0,20 | - | - |
| d-Panthenyltriacetat | 1,00 | 1,00 | - | - |
| Konservierungsmittel | q.s. | q.s. | q.s. | q.s. |
| Parfümöl H1 oder H2 | 1,50 | - | 1,15 | - |
| Parfümöl G2 aus Beispiel 5 | - | 0,90 | - | 0,75 |
| Wasser | Ad 100 | Ad 100 | Ad 100 | Ad 100 |

### Parfümöle für die Transparente Deo-Stifte bzw. Deo-Creme-Stifte

| **Riechstoffe** | **Parfümöl H1** (Vergleich) | **Parfümöl H2** (erfindungsgemäß) |
|---|---|---|
| ALDEHYDE C10 | 0,5 | 0,5 |
| ALDEHYDE C12 MNA 10% DPG | 2,0 | 2,0 |
| MINTONAT | 40,0 | 40,0 |
| MELOZONE | 0,5 | 0,5 |
| VERTOCITRAL | 6,0 | 6,0 |
| DIHYDRO MYRCENOL | 120,0 | 120,0 |
| TETRAHYDRO MYRCENOL | 10,0 | 10,0 |
| AGRUNITRIL | 1,0 | 1,0 |
| ORANGE OIL BRASIL | 10,0 | 10,0 |
| TAMARINE TYPE BASE | 10,0 | 10,0 |
| HEXYL ACETATE | 6,0 | 6,0 |
| ALDEHYDE C14 SO-CALLED | 1,0 | 1,0 |
| TETRAHYDRO LINALOOL | 40,0 | 40,0 |
| DIMETHYL BENZYL CARBINYL BUTYRAT | 5,0 | 5,0 |
| ORANGE FLOWER ETHER | 5,0 | 5,0 |
| PHENYLETHYL ACETATE | 6,5 | 6,5 |
| PHENYLETHYL ALCOHOL | 15,0 | 15,0 |
| GERANIOL SUPER | 30,0 | 30,0 |
| ROSAPHEN^{®} | 25,0 | 25,0 |
| ISODAMASCON^{®} | 0,5 | 0,5 |
| HEDIONE | 300,0 | 300,0 |
| UNDECAVERTOL | 0,5 | 0,5 |
| ALLYL IONONE | 2,5 | 2,5 |
| IONONE BETA | 10,0 | 10,0 |
| EUGENOL | 4,0 | 4,0 |
| ISO E SUPER | 30,0 | 30,0 |
| BRAHMANOLO | 2,0 | 2,0 |
| AMBROXIDE | 2,0 | 2,0 |
| GLOBALIDE^{®} | 12,0 | 12,0 |
| GLOBANONE^{®} | 8,0 | 8,0 |
| MACROLIDE^{®} SUPRA | 45,0 | 45,0 |
| **MESACONSÄUREDIETHYLESTER** | - | **50,0** |
| DIPROPYLENE GLYCOL | 300,0 | 250,0 |
| | 1.000,0 | 1.000,0 |

### BEISPIEL 7: Haarconditioner mit UV-Schutz

| | **Komponente** | **INCI Name** | **Gew.-%** | **Gew.-%** |
|---|---|---|---|---|
| Lanette O | | Cetearyl Alcohol | 4,00 | 4,00 |
| Dragoxat 89 | | Ethylhexyl Isononanoate | 4,00 | 4,00 |
| Emulsiphos | | Potassium Cetyl Phosphate, Hydrogenated Palm Glycerides | 0,50 | 0,50 |
| Natrosol 250 HR | | Hydroxyethylcellulose | 0,25 | 0,25 |
| Neo Heliopan Hydro | | Phenylbenzimidazole Sulfonic Acid | 2,00 | 2,00 |
| L- Arginin | | Arginine | 1,20 | 1,20 |
| Benzophenon-4 | | Benzophenone-4 | 0,50 | 0,50 |
| Neo Heliopan AP | | Disodium Phenyl Dibenzimidazole Tetrasulfonate | 0,50 | 1,00 |
| Edeta BD | | Disodium EDTA | 0,05 | 0,05 |
| Dragocide Liquid | | Phenoxyethanol (and) Methylparaben (and) Butyparaben (and) Ethyparaben (and) Propylparaben | 0,80 | 0,80 |
| Dow Corning 949 Cationic Emulsion | | Amodimethicone, Cetrimonium Chloride, Trideceth-12 | 2,00 | 2,00 |
| Dow Corning 5200 | | Laurylmethicone Copolyol | 0,50 | 0,50 |
| Parfümöl B2 aus Beispiel 2 | | Parfum | 0,95 | - |
| Parfümöl H2 aus Beispiel 6 | | Parfum | - | 1,25 |
| Wasser | | Water (Aqua) | Ad 100 | Ad 100 |

### BEISPIEL 8: Sonnenschutzmilch (W/O)

| **Teil** | **Rohstoffe** | **INCI Bezeichnung** | **Gew.-%** |
|---|---|---|---|
| **A** | Dehymuls PGPH | Polyglyceryl-2 | 3,00 |
| | | Dipolyhydroxystearate | |
| | Bienenwachs 8100 | Beeswax | 1,00 |
| | Monomuls 90-0-18 | Glyceryl Oleate | 1,00 |
| | Zinkstearate | Zinc stearate | 1,00 |
| | Cetiol SN | Cetearyl Isononanoate | 5,00 |
| | Cetiol OE | Dicaprylyl Ether | 5,00 |
| | Tegosoft TN | C12-15 Alkyl Benzoate | 4,00 |
| | Vitamin E | Tocopherol | 0,50 |
| | Solbrol P | Propylparaben | 0,10 |
| | Neo Heliopan^{®} OS | Ethylhexyl Salicylate | 5,00 |
| | Neo Heliopan^{®} AV | Ethylhexyl Methoxycinnamate | 7,50 |
| | Uvinul^{®} T150 | Ethylhexyl Triazone | 1,50 |
| **B** | Wasser, destilliert | Water (Aqua) | Ad 100 |
| | Trilon BD | Disodium EDTA | 0,10 |
| | Glycerin | Glycerin | 5,00 |
| | Solbrol M | Methylparaben | 0,20 |
| | Phenoxyethanol | Phenoxyethanol | 0,70 |
| | Neo Heliopan^{®} AP 10%ige | Disodium Phenyl | 15,00 |
| | Lösung, neutralisiert mit NaOH | Dibenzimidazole Tetrasulfonate | |
| **C** | Parfümöl C2 aus Beispiel 3 | Parfum (Fragrance) | 0,25 |
| | Alpha Bisabolol | Bisabolol | 0,10 |

### Herstellungsverfahren

Teil A: Auf ca. 85 °C erhitzen.
Teil B: Auf ca. 85 °C erhitzen. B zu A geben. Unter Rühren abkühlen lassen.
Teil C: Zugeben und anschließend homogenisieren.

### BEISPIEL 9: Haarfärbungscreme für eine blonde Farbtönung

| **Teil** | **Material** | **Hersteller** | **INCI-Name** | **Gew.-%** |
|---|---|---|---|---|
| **A** | Oxowax | LCW, Frankreich | Cethyl alcohol, Oleyl alcohol, Cetearyl alcohol, Stearic acid | 21,00 |
| | Volpo CS 25 | Croda GmbH, Deutschland | Ceteareth25 | 4,00 |
| | Berol 175 | Brenntag Eurochem GmbH, Deutschland | Laureth-8 | 10,00 |
| | Lanette^{®} E | BAST Personal Care & Nutrition GmbH | Sodium Cetearyl Sulfate | 1,00 |
| | Covaquat 16 | LCW, Frankreich | Polyquaternium-6 | 4,00 |
| | Wasser | | Water (Aqua) | 49,30 |
| **B** | Ammoniak (20% in Wasser) | Merk Eurolab | Ammonia | 10,20 |
| **C** | Parfümöl D2 aus Beispiel 4 | Symrise AG | Parfum | 0,50 |

Teil A, B und C außer Covaquat 16 zusammen mischen und 20 Minuten bei 80 °C erhitzen. Nachdem das Produkt homogen ist abkühlen lassen. Covaquat 16 bei 50 °C dazugeben. Wenn das Produkt anfängt zu verdicken die Rührung stoppen. Bei Raumtemperatur NH₄OH dazugegeben und die Mischung bis Homogenisierung rühren.

### BEISPIEL 10: Kerze

| **Material** | **Gew.-%** |
|---|---|
| Kerzenwachs | 95,00 |
| Parfümöl D2 aus Beispiel 4 | 5,00 |

Das Kerzenwachs schmelzen lassen und rühren. Das Parfümöl dazugeben, gut rühren. In die gewünschte Form eingießen.

### BEISPIEL 11: Parfümöle für Eau De Toilette (10%ig in Ethanol)

| | **J1 (Vergleichsbeispiel)** | **J2 (Erfindungsgemäßes Beispiel)** |
|---|---|---|
| ACETESSIGSAEUREETHYLESTER | 50 | 50 |
| FLORALOZONE 10% DPG | 50 | 50 |
| HEXENOL CIS-3 | 30 | 30 |
| HEXENYLACETAT CIS-3 10% DPG | 10 | 10 |
| LEAFOVERT^{®} | 20 | 20 |
| CYCLOGALBANAT^{®} 10% DPG | 30 | 30 |
| MAGNOLAN | 305 | 305 |
| STYROLYLACETAT | 20 | 20 |
| METHYLANTHRANILAT 10% DPG | 20 | 20 |
| RED BERRIES EXTRACT | 30 | 30 |
| ALDEHYD C14 SOG. EXTRA | 20 | 20 |
| ETHYLMETHYLBUTYRAT-2 1% DPG | 30 | 30 |
| MANZANATE 1% DPG | 50 | 50 |
| APRIFLOREN^{®} | 10 | 10 |
| DAVANAOEL F. PARF. 10% DPG | 30 | 30 |
| CASSIS 345F TYPE BASE | 20 | 20 |
| LILIAL | 100 | 100 |
| CALONE 1 % DPG | 100 | 100 |
| HELIONAL | 200 | 200 |
| FLOROSA | 300 | 300 |
| ETHYLLINALOOL | 470 | 470 |
| ROSENOXID D 10% DPG | 50 | 50 |
| PHENYLETHYLALKOHOL REIN | 20 | 20 |
| CITRONELLOL 950 | 50 | 50 |
| DAMASCENON 10% DPG | 30 | 30 |
| ROSACETAT | 10 | 10 |
| HEDION HC/70 | 1.500,00 | 1.400,00 |
| UNDECAVERTOL | 30 | 30 |
| IONON BETA | 230 | 230 |
| ISO E SUPER NON DISCOLORING | 300 | 300 |
| EVERNYL 10% DPG | 10 | 10 |
| AMBROXIDE | 50 | 50 |
| ETHYLENBRASSYLAT | 1.000,00 | 1.000,00 |
| GLOBALIDE^{®} | 600 | 500 |
| GLOBANONE^{®} | 400 | 400 |
| GALAXOLID PURE | 1.800,00 | 1.800,00 |
| TONALID | 500 | 500 |
| **MESACONSÄUREDIETHYLESTER** | | 200 |
| INDOL FF 10% DPG | 25 | 25 |

Der Akkord mit Mesaconsäurediethylester vermittelt einen natürlichen intensiv birnenartigen Eindruck.

### BEISPIEL 12: Parfümöle für Eau De Toilette (10%ig in Ethanol)

| | **K1 (Vergleichsbei spiel)** | **K2 (Erfindungsgemäßes Beispiel)** |
|---|---|---|
| ACETESSIGSAEUREETHYLESTE R | 10 | 10 |
| HEXENOL CIS-3 | 2,5 | 2,5 |
| HEXENYLACETAT CIS-3 1% DPG | 4 | 4 |
| LEAFOVERT^{®} 10% DPG | 5 | 5 |
| VERTOCITRAL 10% DPG | 4 | 4 |
| PHENYLACETALDEHYD DIMETHYLACETAL 10% DPG | 3 | 3 |
| CYCLOGALBANAT^{®} 10% DPG | 6 | 6 |
| MAGNOLAN | 9 | 9 |
| STYROLYLACETAT | 1 | 1 |
| DIHYDROMYRCENOL | 6 | 6 |
| LINALYLACETAT | 36 | 36 |
| CITRONENOEL ITAL. | 10 | 10 |
| ORANGENOEL ITAL.SUESS ENTF. | 2 | 2 |
| AMAROCIT^{®} | 1 | 1 |
| OXANE 1% DPG | 3 | 3 |
| METHYLANTHRANILAT 10% DPG | 1 | 1 |
| ISOAMYLACETAT | 1 | 1 |
| ALDEHYD C14 SOG | 1,5 | 1,5 |
| DECALACTON GAMMA 10% DPG | 2 | 2 |
| CASSIS BASE 345 BB | 10 | 10 |
| FRAMBINON^{®} | 1,5 | 1,5 |
| ETHYLMALTOL | 50 | 50 |
| BOURGEONAL | 2,5 | 2,5 |
| HELIONAL | 5 | 5 |
| FLOROSA BM / PYRANOL | 55 | 50 |
| HYDROXYCITRONELLAL | 20 | 20 |
| ETHYLLINALOOL | 38 | 33 |
| LINALOOL | 14 | 14 |
| DIMETHYLBENZYLCARBINYLBUT YRAT | 5 | 5 |
| ROSENOXID | 1,5 | 1,5 |
| PHENIRAT^{®} | 3 | 3 |
| PHENYLETHYLALKOHOL | 1 | 1 |
| PHENYLETHYLISOBUTYRAT 10% DPG | 3 | 3 |
| CITRONELLOL 950 | 2 | 2 |
| GERANIOL SUPRA | 3 | 3 |
| NEROL 900 | 2 | 2 |
| CITRONELLYLFORMIAT 10% DPG | 5 | 5 |
| GERANYLFORMIAT SUPRA | 1 | 1 |
| DAMASCENON | 1 | 1 |
| DAMASCON ALPHA 10% DPG | 3 | 3 |
| BENZYLACETAT | 6,5 | 6,5 |
| HEDION HC/30 | 69 | 69 |
| HEXYLZIMTALDEHYD ALPHA | 2 | 2 |
| JASMINLACTON | 1,5 | 1,5 |
| BENZYLSALICYLAT | 32 | 32 |
| HEXENYLSALICYLAT CIS-3 | 13 | 13 |
| DIHYDROIONON BETA | 15 | 15 |
| ISORALDEIN 95 | 8 | 8 |
| ETHYLVANILLIN | 36 | 36 |
| VANILLIN | 19 | 19 |
| ZIMTALKOHOL | 1 | 1 |
| CUMARIN | 4 | 4 |
| AGRUMEX LC | 1 | 1 |
| CASHMERAN | 12 | 12 |
| CEDRAMBER | 12 | 12 |
| ISO E SUPER | 150 | 150 |
| TRIMOFIX O | 15 | 15 |
| PATCHOULIOEL ENTF. DM | 22 | 22 |
| ISOBORNYLCYCLOHEXANOL | 35 | 35 |
| AMBROXIDE | 3 | 3 |
| AMBRETTOLIDE | 8 | 8 |
| MACROLIDE^{®} SUPRA | 22 | 22 |
| GALAXOLID 50% IN IPM | 52,5 | 52,5 |
| HELVETOLIDE | 20 | 20 |
| INDOL FF 10% DPG | 3 | 3 |
| **MESACONSÄUREDIETHYLESTER** | | 10 |
| BENZYLALKOHOL DD | 3 | 3 |

Die Grundnote ist deutlich stärker birnenartig mit Mesaconsäurediethylester.

### BEISPIEL 13: Parfümöl für Eau de Toilette (10%ig in Ethanol)

| | **L1 (Vergleichsbei spiel)** | **L2 (Erfindungsgemäßes Beispiel)** |
|---|---|---|
| ACETESSIGSAEUREETHYLESTE R | 1 | 1 |
| HEXENOL CIS-3 10% DPG | 5 | 5 |
| HEXENYLACETAT CIS-3 10% DPG | 4 | 4 |
| HEXENYLISOBUTYRAT CIS-3 10% DPG | 5 | 5 |
| LEAFOVERT^{®} 10% DPG | 5 | 5 |
| STYROLYLACETAT | 2 | 2 |
| BERGAMOTTOEL ECHT | 5 | 5 |
| LINALYLACETAT | 20 | 20 |
| ORANGENOEL GUINEA | 2 | 2 |
| AMAROCIT^{®} | 6 | 6 |
| BUTYLACETAT 10% DPG | 1 | 1 |
| HEXYLACETAT | 4 | 4 |
| ISOAMYLACETAT 10% DPG | 1 | 1 |
| PRENYLACETAT 10% DPG | 2 | 2 |
| ALDEHYD C14 SOG. EXTRA | 2 | 2 |
| DECALACTON GAMMA | 1,5 | 1,5 |
| MANZANATE 1% DPG | 5 | 5 |
| WEINHEFENOEL GRUEN MAXIMAROME 1% DPG | 5 | 5 |
| ETHYLMALTOL | 10 | 10 |
| MALTOL | 2,5 | 2,5 |
| BOURGEONAL | 1 | 1 |
| CYCLAMENALDEHYD | 1,5 | 1,5 |
| HELIONAL | 5 | 5 |
| FLOROSA | 40 | 40 |
| HYDROXYCITRONELLAL | 5 | 5 |
| ETHYLLINALOOL | 20 | 20 |
| LINALOOL | 15 | 15 |
| DIMETHYLBENZYLCARBINYLACE TAT | 2 | 2 |
| PHENYLETHYLALKOHOL REIN | 5 | 5 |
| CITRONELLOL 950 | 8 | 8 |
| GERANIOL SUPRA | 5 | 5 |
| CITRONELLYLACETAT EXTRA | 5 | 5 |
| GERANYLACETAT PUR | 10 | 10 |
| NERYLACETAT | 5 | 5 |
| DAMASCENON TOTAL 10% DPG | 5 | 5 |
| DAMASCON ALPHA 10% DPG | 6 | 6 |
| BENZYLACETAT FG | 2 | 2 |
| HEDION | 120 | 100 |
| HEXYLZIMTALDEHYD ALPHA | 6 | 6 |
| JASMON CIS 10% DPG | 3 | 3 |
| BENZYLSALICYLAT | 30 | 30 |
| HEXENYLSALICYLAT CIS-3 | 10 | 10 |
| HEXYLSALICYLAT | 30 | 30 |
| UNDECAVERTOL | 2 | 2 |
| ISORALDEIN 95 | 10 | 10 |
| ISOEUGENOLACETAT 10% DPG | 3 | 3 |
| ETHYLVANILLIN 10% DPG | 1 | 1 |
| VANILLIN | 3 | 3 |
| ZIMTALKOHOL | 2 | 2 |
| CUMARIN | 3 | 3 |
| AGRUMEX HC | 5 | 5 |
| CEDERNHOLZOEL VIRGINIA | 10 | 10 |
| AMBERWOOD@ F | 10 | 10 |
| CASHMERAN | 10 | 10 |
| ISO E SUPER NON DISCOLORING | 85 | 85 |
| POLYSANTOL (MYSANTOL) | 5 | 5 |
| AMBROXIDE | 8 | 8 |
| AMBRETTOLIDE | 10 | 10 |
| GLOBALIDE^{®} | 10 | 10 |
| MACROLIDE^{®} SUPRA | 50 | 50 |
| GALAXOLID 50% IN IPM | 115 | 105 |
| HELVETOLIDE | 10 | 10 |
| **MESACONSÄUREDIETHYLESTER** | | 30 |
| DIPROPYLENGLYCOL | 19,5 | 19,5 |

Stärkerer fruchtiger Birnengeruch mit Mesaconsäurediethylester.

### BEISPIEL 14: Sensorische und physikochemische Beurteilung von Mesaconsäurediethylester

| **Test** | **Skala** | **Mesaconsäurediethylester** |
|---|---|---|
| Schwellenwert in DEP ¹⁾ | 1-13 | 6,3 |
| Wirkung auf Löschpapier ¹⁾ | 1-9 | 5 |
| Verweildauer auf Löschpapier ¹⁾ | 1-9 | 2,7 |
| Schwellenwert in der Luft ¹⁾ | 5-13 | 6,4 |
| Schwellenwert in der Luft ¹⁾ | ppm | 0,047 |
| Intensität in der Luft ¹⁾ | 1-9 | 4 |
| Diffusität | 1-9 | 7,2 |
| Blühen aus Wasser ¹⁾ | 1-9 | 6 |
| Anosmie ²⁾ | % | 0 |
| Wasserlöslichkeit ³⁾ | 1-9 | 6,2 |
| Polarität ⁴⁾ | 1-9 | 3,4 |
| Volatilität ⁵⁾ | 1-9 | 6,4 |
| Geschätzte Stabilität Alkalien ⁶⁾ | | |
| Geschätzte Stabilität Säuren ⁶⁾ | | |
| Geschätzte Stabilität gegen Oxidation ⁶⁾ | | |
| Reduktion Badezimmergeruch ⁸⁾ | 0-7 | 4,1 |
| Duftintensität im Badezimmer ⁹⁾ | 1-9 | 5,4 |
| Reduktion Küchengeruch ⁸⁾ | 0-7 | 4,5 |
| Duftintensität in der Küche ⁹⁾ | 1-9 | 4,9 |
| Reduktion Schweißgeruch ⁸⁾ | 0-7 | 4,2 |
| Duftintensität Schweiß ⁹⁾ | 1-9 | 5,1 |
| Reduktion Rauchgestank ⁸⁾ | 1-7 | 3,5 |
| Duftintensität Rauch ⁹⁾ | 1-9 | 4,5 |

### In diesem Zusammenhang bedeuten:

(1) Je höher der Wert desto höher die geruchliche Leistung
(2) Prozentsatz von Probanden, die den Geruch nicht wahrnehmen können
(3) Je höher der Wert desto höher die Wasserlöslichkeit
(4) Je höher der Wert desto niedriger die Polarität
(5) Je höher der Wert desto höher die Flüchtigkeit, je niedriger der Wert desto höher die Haftung
(6) Einteilung: hoch - moderat - gering
(7) Standard: Linalool (Wert = 5)
(8) Skala 1 (starker unangenehmer Geruch) bis 7 (kein unangenehmer Geruch)
(9) Skala 1 (kein Geruch) bis 9 (starker Geruch)

### BEISPIEL 15: Stabilitätsuntersuchungen

Mesaconsäurediethylester (MSDEE) wurde alleine sowie als Bestandteil verschiedener Rezepturen über 24 bzw. 72 Stunden unter drastischen Bedingungen bei 60 °C und 5 bar Druck gelagert. Anschließend wurde die verbliebene Menge an Wirkstoff bestimmt. Die Ergebnisse sind in der nachfolgenden **Tabelle 3** zusammengefasst:

**Tabelle 3: Lagerstabilität**

| **Produkt** | **Anteil MSDEEE [Gew**.-**%]** | **Nach 24 h [%]** | **Nach 72 h [%]** |
|---|---|---|---|
| MSDEE | 100 | 98 | 98 |
| Eau de Toilette | 4,5 | 99 | 99 |
| Deodorant | 0,5 | 94 | 48 |
| Shampoo | 0,5 | 100 | 100 |
| Weichspüler | 0,5 | 92 | 89 |

## Patentansprüche

1. Verfahren zur Herstellung eines Mono- und/oder Diesters der Mesaconsäure, umfassend die folgenden Schritte:
(i) Umsetzung von Itaconsäure mit Essigsäureanhydrid unter Erhalt von Itaconsäureanhydrid;
(ii) Isomerisierung von Itaconsäureanhydrid zu Citraconsäureanhydrid;
(iii) Veresterung von Citraconsäureanhydrid mit einem aliphatischen, araliphatischen oder aromatischen Alkohol mit 1 bis 10 Kohlenstoffatomen oder Diol mit 1 bis 5 Hydroxylgruppen unter Erhalt eines Mono- und/oder Diesters der Citraconsäure; und
(iv) Umlagerung des Mono- und/oder Diesters der Citraconsäure zu einem Mono- und/oder Diester der Mesaconsäure.

2. Verfahren zur Herstellung eines Mono- und/oder Diesters der Mesaconsäure nach Anspruch 1, wobei in Schritt (ii) ein Lösungsmittelsystem verwendet wird, umfassend ein hoch siedendes Lösemittel, bevorzugt ein Polyalkylenglykol (PAG), mit einem Siedepunkt von größer 150 °C und weiterhin umfassend ein Co-Lösungsmittel mit einem Siedepunkt von zwischen 90 und 120 °C, bevorzugt Dioxan.

3. Verfahren zur Herstellung eines Mono- und/oder Diesters der Mesaconsäure umfassend die folgenden Schritte:
(i) Bereitstellen einer Lösung enthaltend mindestens einen Mono- und/oder Diester der Citraconsäure und gegebenenfalls ein Lösungsmittel;
(ii) Versetzen der Lösung mit Jod;
(iii) Erhitzen der jodhaltigen Lösung auf etwa 170 bis 200 °C und
(iv) gegebenenfalls destillative Aufreinigung des resultierenden Produktes.

4. Verfahren nach Anspruch 1 bis 3, **dadurch gekennzeichnet, dass** der Mono- und/oder Diester der Mesaconsäure (E)-2-Methyl-but-2-endicarbonsäureester, bevorzugt (E)-2-Methyl-but-2-endicarbonsäure-diethylester ist.
